(19) Europäisches Patentamt
European Patent Office
Office européen des brevets

(11) **EP 3 434 323 B1**

(12) **EUROPÄISCHE PATENTSCHRIFT**

(45) Veröffentlichungstag und Bekanntmachung des Hinweises auf die Patenterteilung:
**08.04.2020 Patentblatt 2020/15**

(51) Int Cl.:
*A61N 1/36* *(2006.01)*      *A61N 1/04* *(2006.01)*

(21) Anmeldenummer: **18186033.9**

(22) Anmeldetag: **27.07.2018**

(54) **VORRICHTUNG ZUR FUNKTIONALEN ELEKTRISCHEN STIMULATION UNTER WASSER**

DEVICE FOR FUNCTIONAL ELECTRICAL STIMULATION UNDER WATER

DISPOSITIF DE STIMULATION ÉLECTRIQUE FONCTIONNELLE SOUS L'EAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.07.2017 DE 102017007229**
**21.03.2018 DE 102018106634**

(43) Veröffentlichungstag der Anmeldung:
**30.01.2019 Patentblatt 2019/05**

(73) Patentinhaber: **Voigt, Hanno**
**16536 Ahrensfelde (DE)**

(72) Erfinder: **Voigt, Hanno**
**16536 Ahrensfelde (DE)**

(74) Vertreter: **Hertin und Partner**
**Rechts- und Patentanwälte PartG mbB**
**Kurfürstendamm 54/55**
**10707 Berlin (DE)**

(56) Entgegenhaltungen:
**WO-A1-90/04955          US-A1- 2014 163 444**
**US-A1- 2014 277 220     US-A1- 2017 080 207**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung und ein System zur sensorischen Stimulation oder Erzeugung einer Muskelbewegung im Wasser durch elektrische Stimulation von Nerven und/oder Muskeln, wobei die Vorrichtung einen Stimulator, mindestens eine Elektrode zur Übertragung elektrische Pulse auf die Haut sowie mindestens ein Kabel zur Verbindung des Stimulators mit der mindestens einen Elektrode umfasst und diese wasserdicht ausgebildet sind, wobei die Steuereinrichtung zur Stimulation einer Schwimmbewegung konfiguriert ist. Offenbart werden weiterhin Verwendungen und Verfahren zur sensorischen Stimulation oder Erzeugungen von Muskelbewegungen im Wasser durch elektrische Stimulation von Nerven und/oder Muskeln.

Hintergrund der Erfindung und Stand der Technik

Die Erfindung liegt auf dem Gebiet der Freizeit oder Medizintechnik und ist mit besonderem Vorteil in der Rehabilitationsmedizin anwendbar. Es sind auch Anwendungen auf anderen Gebieten der Medizintechnik und insbesondere der Sporttechnik denkbar.

Eine Verletzung der Wirbelsäule mit einhergehenden Lähmungen der unteren Extremitäten bedeutet in vielen Fällen eine starke Einschränkung der körperlichen Aktivität und der Gesundheit für die Betroffenen. In Abhängigkeit von der Höhe und dem Schweregrad der Verletzung bedeutet dies oft eine funktionelle Einschränkung von verschiedenen Körperfunktionen. Darüber hinaus steht die körperliche Inaktivität aufgrund der Verletzung oft im starken Kontrast zum Zustand vor der Verletzung, besonders für junge Patienten.

Die Teilnahme an sportlichen und therapeutischen Aktivitäten ist oft stark eingeschränkt nach einer Querschnittslähmung durch den Verlust der willkürlichen Motorfunktion und eine ineffiziente Temperaturregulierung der betroffenen Extremitäten, autonomer Dysfunktion sowie eine früh beginnende Muskelermüdung.

Darüber hinaus wird für eine Teilnahme an sportlichen und therapeutischen Aktivitäten speziell angepasstes Equipment und Hilfspersonen benötigt. Trotz all dieser Hindernisse kann gerade die sportliche und therapeutische Aktivität nach einer Querschnittslähmung jedoch zu einer Reduktion von Begleiterkrankungen und zu einer Erhöhung des emotionalen Wohlbefindens der Betroffen beitragen.

In den meisten Fällen führt eine Querschnittslähmung zur kompletten oder inkompletten Lähmung der unteren Extremitäten, so dass ein effektives und sicheres Training der unteren Extremitäten nur eingeschränkt möglich ist. Daher werden weitestgehend Trainingsübungen der oberen Extremitäten empfohlen wie Armkurbelergometer, Rollstuhlergometer oder Schwimmen. All diese Übungen können die physische Fitness bei regelmäßigem Training um bis zu 25% verbessern (Nash *et al.* 2005)

Funktionelle Elektrostimulation (FES) wird besonders beim FES-Fahrradfahren oder FES-Rudern erfolgreich eingesetzt. Dabei werden die entsprechenden Muskeln für Kniestreckung und -beugung bzw. Hüftstreckung und -beugung in Abhängigkeit vom Kurbelwinkel (Fahrradfahren) oder durch einen Zugschalter beim Rudern ausgelöst. Durch die Kombination von Arm- und Beintraining kann ein deutlich höherer Trainingseffekt erzielt werden. Darüber hinaus konnte in einigen Studien eine Verbesserung der Durchblutung und Knochendichte der unteren Extremitäten beobachtet werden (Newham *et al.* 2007). Allerdings werden speziell angepasstes Equipment und/oder Hilfspersonen benötigt. Zudem stellen sich die Erfolge in einigen Fällen nicht oder nicht in dem gewünschten Maße ein.

Aus der WO 90/04955 ist eine Vorrichtung zur Erhöhung der Wärmezu- und abfuhr bekannt, wobei zur Erhöhung des Blutflusses auf der äußeren Oberfläche der Haut elektrische Pulse mittels Elektroden appliziert werden. Zu diesem Zweck wird ein Pulsgenerator vorgeschlagen, welcher in bevorzugten Ausführungsformen in einem wasserdichten Gehäuse eingeschlossen wird. Eine funktionelle Elektrostimulation (FES) mittels der Elektroden zur Stimulation von Bewegungsmustern wird nicht offenbart.

Es besteht mithin ein erhöhter Bedarf an alternativen Methoden zur Nerven- oder Muskelstimulation, welche sowohl zum Bewegungstraining in der Rehabilitationsmedizin als auch im Freizeitbereich einsetzbar ist.

Aufgabe der Erfindung

Aufgabe der Erfindung war es eine Vorrichtung und ein Verfahren bereitzustellen, welches die Nachteile des Standes der Technik beseitigt. Eine weitere Aufgabe der Erfindung war es Vorrichtungen oder Verfahren bereitzustellen, welche sich durch einfache Anwendbarkeit auszeichnen und zu starker Verbesserung der Bewegungsfähigkeit führen.

Zusammenfassung der Erfindung

Erfindungsgemäß wird die Aufgabe durch den unabhängigen Anspruch gelöst. Die abhängigen Patentansprüche stellen bevorzugte Ausführungsformen Erfindung dar.

Die Erfindung betrifft eine Vorrichtung zur Erzeugung einer Muskelbewegung im Wasser durch elektrische Stimulation von Nerven und/oder Muskeln, umfassend einen Stimulator mit einer Spannungsquelle und einer Steuereinrichtung, mindestens eine Elektrode zur Befestigung an einer Haut, und mindestens ein Kabel, welches den Stimulator mit der Elektrode verbindet, wobei der Stimulator, die mindestens eine Elektrode und das mindestens ein Kabel wasserdicht ausgebildet sind, die Steuereinrichtung konfiguriert ist an der mindestens einen Elektrode elektrische Pulse zur Übertragung auf die Haut zu erzeugen und wobei die Steuereinrichtung konfiguriert ist zur Stimulation einer Schwimmbewegung..

Besonders bevorzugt sind auch die Verbindungen zwischen Stimulator, Elektrode und Kabel, beispielsweise Steckverbindungen wasserdicht ausgebildet.

Die Vorrichtung liefert ausgezeichnete Trainingsergebnisse für eine funktionelle Stimulation einer Muskelbewegung unter Wasser als Schwimmtraining. Im Stand der Technik waren lediglich Vorrichtungen oder Verfahren bekannt, welche FES zum Bewegungstraining außerhalb des Wassers offenbaren.

Erfindungsgemäß wurde erkannt, dass ein Bewegungstraining unter Wasser ggü. bekannten Ergometertraining oder FES-Fahrradfahren eine Reihe von Vorteilen aufweist. Die Vorteile sind sowohl physiologisch als auch psychologischer Natur.

Zum einen kann im Wasser ein Zustand der Schwerelosigkeit erreicht werden, so dass kaum Hilfsmittel zur Stabilisierung oder Verhinderung von Druckstellen notwendig sind. Weiterhin kann jede Extremität unabhängig bewegt und trainiert werden. Die Freiheitsgrade sind im Wasser deutlich erhöht. Auch können sich Paraplegiker im Wasser sehr ähnlich bewegen wie nicht Betroffene und sind nach einer Schulung oft im Stande ohne Hilfsperson oder Assistenz das Training auszuführen. In Studien konnte bereits eine Verbesserung der Lungenfunktion im Vergleich von Land- zu Wassertraining festgestellt werden (Jung *et al.* 2014).

In der Fachwelt wurde FES-Bewegungstraining im Wasser weder diskutiert noch angeregt. Vielmehr setzte die Fachwelt voraus, dass eine FES mit effektiver Nerven- oder Muskelstimulation stets außerhalb von Wasser durchzuführen sei. Die Erfindung stellt somit einer Abkehr von bekannten Ansätzen und dem Stand der Technik dar, welche überraschender Weise ausgezeichnete Ergebnisse liefert. Diese betrifft sowohl die zuverlässige Stimulation von Muskelbewegungen, als auch den sich einstellenden Trainingserfolg.

Probanden, welche die erfindungsgemäße Vorrichtung anwenden, berichten von einer Steigerung des Wohlbefindens, welches nicht durch eine Fremdeinwirkung oder gar Schmerzempfindung bestimmt ist. Stattdessen wurde die Muskel- oder Nervenstimulation unter Wasser als besonders natürlich und angenehm empfunden Auch Trainingserfolge stellten sich durch häufige und regelmäßige Anwendung der Vorrichtung zuverlässig ein. Aufgrund der Anwendung im Wasser kann den Trainierenden einen Spaß vermittelt werden, wodurch sich die Nutzungsbereitschaft und -dauer signifikant erhöht.

Die Trainingserfolge betrafen einerseits die Rehabilitation von bewegungseingeschränkten Probanden. Andererseits konnten auch bei körperlich gesunde Probanden eine deutliche verbesserte physische Fitness erreicht werden. Diese zeigte sich nicht nur in gesteigerter Muskelkraft und -ausdauer, sondern auch in einer verbesserten Bewegungskoordination, welche auf die besondere Anregung unter Wasser zurückführbar war.

Überraschenderweise ließen sich auch neue Bewegungsmuster durch die FES-Vorrichtung zügig und nachhaltig erlernen. Im Sinne der Erfindung bezeichnen Probanden bevorzugt Nutzer der Vorrichtung, wobei es sich bevorzugt um Menschen mit oder ohne körperliche Bewegungseinschränkung handeln kann.

Die vorteilhafte Anwendbarkeit der Vorrichtung zur Erzeugung einer Muskelbewegung im Wasser durch elektrische Stimulation von Nerven und/oder Muskeln wird erfindungsgemäß durch die wasserdichte Ausgestaltung des Stimulators, der mindestens einen Elektrode und des Kabels ermöglicht.

Im Sinne der Erfindung bezeichnet ein Stimulator bevorzugt eine Vorrichtung oder ein Gerät zur steuerbaren Generation elektrischer Pulse. Zu diesem Zweck weist ein Stimulator bevorzugt eine Spannungsquelle (auch als pulserzeugende Einheit bezeichenbar), welche durch eine Steuereinrichtung geregelt wird. Bevorzugt ist die Spannungsquelle mit einer Batterie oder einem Akkumulator verbunden, wodurch der Stimulator ohne Kabel für eine externe Energiequelle am Körper betrieben werden kann. Geeignete Batterien umfassen beispielsweise Alkali-Mangan-Batterien, Zinkchlorid-Batterien oder Zink-Kohle-Batterien. Als Akkumulatoren eignen sich beispielsweise Lithium-Ionen-Akkumulatoren, Natrium-Ionen-Akkumulatoren oder NiCd - Nickel-Cadmium-Akkumulatoren.

Geeignete Puls-erzeugenden Einheiten für FES sind dem Fachmann hinreichend bekannt. Zur Bereitstellung einer Spannungsquelle zur Generation der elektrischen Pulse können auch kommerzielle erhältliche Produkte wie beispielsweise der RehaMove3 Science von der Hasomed GmbH genutzt werden.

Das Muster, welches mit der Spannungsquelle oder Pulserzeugende Einheit erzeugt wird, wird durch die Steuereinrichtung festgelegt.

Im Sinne der Erfindung ist die Steuereinrichtung bevorzugt ein Prozessor, bevorzugt ein Mikroprozessor oder Prozessorchip, ein integrierter Schaltkreis und/oder ein Mikrokontroller, welcher konfiguriert ist, um die Stromstärke, Pulsbreite und Stimulationsfrequenz der elektrischen Pulse gemäß vorgegebener Parameter zu regulieren. Die vorgegebenen Parameter können auf einem Datenspeicher der Steuereinrichtung hinterlegt sein, sodass die Stimulation gemäß eines festlegten Musters erfolgt.

Es kann aber auch bevorzugt sein, dass die vorgegebenen Werte an die Steuereinrichtung über eine Kommunikationsschnittstelle bereits gestellt werden. Beispielsweise kann die Steuereinrichtung mit einer Bedieneinheit verbunden sein, welcher nach Auswahl durch den Nutzer mittels einer verkabelten oder kabellosen Verbindung die Parameter an die Steuereinrichtung übermittelt. Besonders bevorzugt umfasst die Steuereinrichtung eine kabellose Kommunikationsschnittstelle beispielsweise auf Basis von WLAN, Bluetooth oder anderen Kommunikationsprotokollen. Die Kommunikationsschnittstellen der Steuereinrichtung verfügen entsprechend bevorzugt über eine Empfänger- und Sendereinheit, welche den drahtlosen Austausch über diese Kommunikationsprotokolle ermöglichen. Eine drahtlose Kommunikation ist besonders bevorzugt für die Einbindung mobiler Endgeräte oder smart devices.

Die Vorrichtung ist dadurch gekennzeichnet, dass der Stimulator wie auch das Kabel und die Elektroden wasserdicht ausgebildet sind. Im Sinne der Erfindung meint "wasserdicht" bevorzugt, eine Wasserdichtigkeit, welche sicherstellt, dass bei Verwendung der Vorrichtung vollständig unterhalb von Wasser für länger 1h keine Funktionsbeeinträchtigung zu verzeichnen ist. Bevorzugt liegt eine Wasserdichtigkeit der Komponenten vor, bei welcher der Eindringwert von Wasser unterhalb von 50 μg/min, bevorzugt unterhalb von 30 μg/min, besonders bevorzugt unterhalb von 10 μg/min liegt, sofern die Vorrichtung sich unter Wasser bevorzugt in einer Wassertiefe von 1 m befindet.

Um den Stimulator wasserdicht auszugestalten, können verschiedene Konstruktionsweisen vorgesehen sein. Der Stimulator kann beispielsweise durch einen wasserdichten Überzug oder Manschette vollständig ummantelt werden. Auch kann es bevorzugt sein den Stimulator in einem wasserdichten Gehäuse einzuschließen. Bevorzugt ist das wasserdichte Gehäuse aus Kunststoff, bevorzugt transparentem Kunststoff gefertigt. Vorteilhafterweise stellt ein Kunststoffgehäuse eine wasserdichte Ummantelung des Stimulators sicher, ohne die optionale Kommunikation des Stimulators mit einem mobilen Endgerät zu behindern.

Die Applikation der durch den Stimulator genierten elektrischen Pulse erfolgt über die mindestens eine Elektrode auf die Haut. Diese kann bipolar ausgeprägt sein. Bei monopolaren Elektroden muss ebenfalls die mindestens eine Elektrode auf der Haut bzw. am Körper angebracht sein. Zur Erzeugung eines Stromflusses kann eine Gegenelektrode frei im Wasser, am Gehäuse des Stimulators oder ebenfalls am Körper angebracht sein. Im Stand der Technik ist es bekannt für FES Anwendungen bevorzugt Oberflächenelektroden zu verwenden, welche flächig auf die Haut einen elektrischen Kontakt vermitteln. Geeignete medizinische Elektroden sind hinreichend bekannt und bevorzugt flexibel ausgestaltet, um auch bei der Bewegung der menschlichen Haut den Kontakt nicht zu verlieren. Als Standardelektroden werden beispielsweise mehrfach verwendbare Hydrogel-Elektroden mit textiler Beschichtung der Oberseite oder Silikonelektroden verwandt mit typischen Größen von 2-20 cm². Versuche haben jedoch gezeigt, dass Standardelektroden, wie u.a. die Silikonelektroden der Firma AAM GmbH (Artikelnummer Z 0077) nicht ohne Funktionsbeeinträchtigung für FES unter Wasser eingesetzt werden können. Chloriertes Schwimmwasser weist typischerweise eine elektrische Leitfähigkeit von 2,5-3 mS/cm (milli Siemens pro Zentimeter) und somit einen Widerstand von ca. 330-400 Ohm auf. Eine Stimulation mittels solcher Standardelektroden führt daher zu Schleichströmen und Kurzschluss zwischen den Elektroden.

Überraschenderweise konnten derartige Standardelektroden auf einfache Weise abgedichtet werden. Beispielsweise können die Elektroden von einer wasserdichten Manschette ummantelt werden, mit Hilfe derer die Elektroden auf der Haut angebracht werden. Für eine Stimulation des *Musculus quadriceps femoris* eignen sich z.B. Manschetten für den Oberschenkel, welche die Elektroden wasserdicht abdecken.

In einer bevorzugten Ausführungsform ist die Vorrichtung dadurch gekennzeichnet, dass die mindestens eine Elektrode mittels einer sprühbaren Polymerlösung wasserdicht versiegelt wurde. Die sprühbare Polymerlösung ist bevorzugt eine Mischung aus einem flüchtigen Lösungsmittel und einem hydrophoben Polymer, welche mittels bekannter Sprühtechniken aufgesprüht werden kann. Nach dem Aufsprühen auf die Elektrode verdunstet das Lösungsmittel, sodass eine hydrophobe Polymerschicht auf der Elektrode zurückbleibt. Diese versiegelt die Elektrode auf überraschend gute Weise gegenüber dem Eindringen von Feuchtigkeit und Wasser. Als Polymere eignen sich beispielsweise Polyvinylpyrrolidon, Nitrocellulose, Ethylcellulose, Poly(methylacrylat-isobuten-monoisopropylmaleat, oder Cyanacrylate. Besonders bevorzugte ist das die sprühbare Polymerlösung ein Sprühpflaster oder ein Sprühverband ist. Diese sind zum einen kostengünstig kommerziell erhältlich und zum anderen äußerst Anwenderfreundlich bei ausgezeichneten Wasserabdichtungseigenschaften.

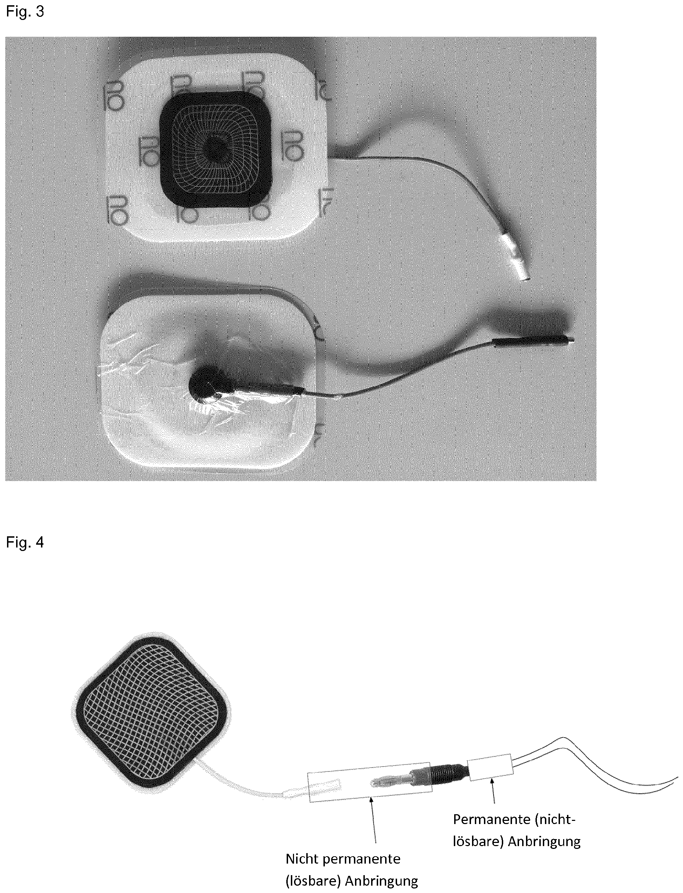

In einer weiteren bevorzugten Ausführungsform werden wasserdichte Elektroden verwandt, welche in der US 7697999 B2 beschrieben werden und deren Inhalt hiermit durch Verweis vollständig einbezogen wird. Die bevorzugten wasserdichten Elektroden umfassen ein leitfähiges flexibles Element mit einer Oberseite und einer Unterseite, einem Konnektor im Kontakt mit dem leitfähigen flexiblen Element zur Herstellung einer elektrischen Verbindung mit einem Stimulator, eine nicht-leitende flexiblen Folie, welche über der Oberseite des leitfähiges flexibles Element angeordnet ist, wobei die nicht-leitfähige Folie eine größere Abmessung als das leitfähige flexible Element aufweist, sodass eine Überlappung entsteht, sowie bevorzugt einen mittigen Kleber zur Verbindung der nicht-leitenden flexiblen Folie mit dem leitfähiges flexiblen Element. Durch die Wasserdichtheit der Elektroden wird die weitere Funktion der Elektroden nach Baden, Duschen und Schwimmen gewährleistet. Die funktionale Verwendung der Elektroden in der erfindungsgemäßen Vorrichtung im Wasser, d.h. die Applizierung elektrischer Impulse, wird bevorzugt eine wasserdichte Klebefolie, z.B. Tegaderm von 3M, über der Verbindung von Elektrode und Kabel aufgebracht werden. Hierdurch kann auch eine Wasserdichtigkeit der Steckverbindung gewährleistet werden. Ein Beispiel einer derart besonders bevorzugten wasserdichten Elektrode wird in Fig. 3 und der dazugehörigen Beschreibung offenbart.

Für die FES unter Wasser haben sich wasserdichten Elektroden mit einer Fläche des leitfähigen Elementes (bspw. einer metallischen Drahtanordnung) von 10 cm$^2$ bis 30 cm$^2$ als besonders vorteilhaft erwiesen. Abmaße für die Abdeckung durch eine nicht-leitende äußere Folie von 50 cm$^2$ - 130 cm$^2$ zeigen ausgezeichnete Ergebnisse in Bezug auf die Wasserdichtigkeit, ohne den Tragekomfort einzuschränken.

In einer weiteren bevorzugten Ausführungsform, handelt es sich bei wasserdichten Elektroden im Sinne der Erfindung, um Elektroden, welche auf einer Seite leitend sind und auf der abgewandten Seite nicht leitend sind. Bei Verwendung der Vorrichtung zur FES unter Wasser werden die Elektroden mit der leitenden Seite am Körper angebracht. Die dem Körper abgewandte Seite ist nicht elektrisch leitend sein. Durch einen Anpressdruck der Elektroden, z.B. durch ein Kleidungsstück oder eine Manschette, kann ein Stimulationseffekt erzielt werden, welcher mit Elektroden, welche komplett wasserdicht ausgestaltet sind, vergleichbar ist. Im Sinne der Erfindung liegt somit bevorzugt eine wasserdichte Elektrode vor, wenn bei Verwendung der Vorrichtung zur FES unter Wasser an einem Körper eine Interferenz von Wasser mit der Stimulation verhindert werden kann. Die Ausführungsform der Elektroden, bei welcher nur eine Seite elektrische leitend ist, während die andere Seite nicht elektrisch leitend ist, zeichnet sich durch eine besonders einfache Anwendbarkeit aus. Insbesondere lassen Sich ausgezeichnete Ergebnisse mit leitfähigen Gummi- oder Silikonelektroden erzielen.

Zur Verbindung des Stimulators mit den Elektroden umfasst die Vorrichtung erfindungsgemäß wasserdichte Kabel. Stecker oder Steckverbindungen mit denen die Kabel an den Simulator und an die Elektroden angeschlossen werden, sind ebenfalls bevorzugt wasserdicht ausgestaltet.

In einer bevorzugten Ausführungsform ist die Vorrichtung dadurch gekennzeichnet, dass die Verbindung der mindestens einen Elektrode mit dem mindestens einen Kabel durch ein von einem Silikonschlauch umfassten Stecker erfolgt.

Vorteilhafterweise ermöglicht die Ummantelung mittels eines Silikonschlauches eine lösbare wasserdichte Anbringung, welche ein An- oder Abklemmen der Elektroden auf einfache Weise gewährleistet. Zur Abdichtung der Steckverbindungen können zwar auch andere Mittel wie Muffen oder dergleichen verwendet werden, der Silikonschlauch zeichnet sich jedoch durch Flexibilität, Wasserdichtigkeit und Handhabbarkeit in besonderem Maße aus.

Die Standardstecker kommerzieller erhältlicher Stimulatoren sind oftmals auch auf ihrer rückseitigen Verbindung zum Kabel nicht wasserdicht. Somit trat in Versuchen im hinteren Bereich des Steckers Wasser in die Verbindung ein. Eine permanente Verklebung mittels Schrumpfschlauch oder Heißkleber kann dies auf besonders einfache und effektive Weise verhindern.

Durch die vorgenannten bevorzugten Ausführungsformen konnten für die Unterwasseranwendung eine besonders taugliche FES Vorrichtung bereitgestellt werden, welche durch besonders wasserdichte Stimulatoren, Elektroden, Kabel sowie Verbindungen zwischen diesen gekennzeichnet sind. Hierdurch können zuverlässig elektrische Pulse unter Wasser auf die Haut eines Menschen appliziert werden, welche zur sensorischen Stimulation oder zur Erzeugung einer Muskelbewegung führen.

In einer bevorzugten Ausführungsform ist die Vorrichtung dadurch gekennzeichnet, dass die elektrischen Pulse mono- oder biphasisch sind und eine Energie von 10 mJ nicht überschreiten, wobei die Pulse bevorzugt eine Stromstärke zwischen 0-120 mA und eine Pulsbreite zwischen 0 und 1000 $\mu$s aufweisen.

Monophasische elektrische Pulse bezeichnen bevorzugt Pulse gleicher positiver oder negativer Amplitude, wohingegen biphasische Pulse dadurch gekennzeichnet sind, dass auf einen Puls ein invertierter Puls gleicher Energie aber entgegengesetzter Amplitude folgt.

Mit den vorgenannten Pulsenergien bzw. Stromstärken und Pulsbreiten kann überraschenderweise auch unter Wasser eine effektive Stimulation der Muskel oder Nerven erreicht werden. Für eine Stimulation zur Anregung einer Muskelbewegung, insbesondere der Beine, haben sich Energiewerte zwischen 2 mJ und 4 mJ als besonders vorteilhaft erwiesen.

Der Abstand zwischen zwei elektrischen Pulsen beschreibt die Periodendauer oder Frequenz der elektrischen Pulse. Mit höherer Frequenz steigt die Kraft, die ein Muskel erzeugen kann. Allerdings ermüdet der Muskel auch deutlich schneller. Besonders gute Ergebnisse konnten mit Frequenzen zwischen 10 und 100 Hz erzielt werden. Während der Stimulation eines Muskels wird in der Regel eine Pulsserie appliziert. Um eine Kontraktion eines Muskels unter Wasser zu erzeugen, hat sich beispielsweis eine Stimulationsdauer unter Applikation mehrere Pulse von 0,2 - 5 Sekunden als ausreichend herausgestellt.

Mit deutlich geringerer Energieleistung von bevorzugt 0.5 mJ und 1 mJ ist eine sensorische Stimulation unter Wasser möglich. Diese führt nicht zu einer unmittelbaren Muskelbewegung, sondern dem subjektiven Eindruck einer Muskelbewegung. Überraschenderweise kann durch eine sensorische Stimulation ebenfalls die Beweglichkeit und das Wohlbefinden der Probanden deutlich gesteigert werden.

In einer bevorzugten Ausführungsform der Erfindung umfasst die Vorrichtung Befestigungsmittel zur Positionierung der mindestens einen Elektrode (A) auf der Haut eines Beines eines Menschen oberhalb des *Musculus quadriceps femoris* und/oder der ischiokruralen Muskulatur umfasst, wobei es besonders bevorzugt ist, dass die Vorrichtung mindestens zwei Elektroden umfasst sowie Befestigungsmittel zur Positionierung der mindestens zwei Elektroden auf der Haut jeweils eines Beines eines Menschen oberhalb des *Musculus quadriceps femoris* und/oder der ischiokrurale Muskulatur.

Die Befestigungsmittel definieren sich funktionell, um die vorgenannte Positionierung zu ermöglichen. Beispielsweise kann das Befestigungsmittel eine Manschette sein, welche an die Größe eines menschlichen Oberschenkels angepasst ist und somit die geforderte Positionierung ermöglicht. Auch die Länge der Kabel zwischen dem Stimulator und der Elektrode, ist für die bevorzugte Ausführungsform an die Positionierung angepasst. Die Befestigungsmittel zur Positionierung der Elektroden auf den vorgenannten Muskeln stellen somit strukturelle Merkmale dar, welche die bevorzugte Ausführungsform der Vorrichtung kennzeichnen.

Der *Musculus quadriceps femoris* (im Folgenden auch als Quadrizeps abgekürzt) ist ein aus vier Muskelköpfen bestehender Skelettmuskel auf der Vorderseite des Oberschenkels. Die Anbringung der Elektrode erfolgt bevorzugt diesem Bereich. Eine Stimulation der Muskelpartie führt zu einer Extension des Kniegelenkes. Im Gegensatz dazu ist die *ischiocrurale* Muskulatur oder rückseitige Oberschenkelmuskulatur (im Englischen *hamstring*) eine Gruppe von Muskeln, welche zu einer Kniebeugung führt.

Die Ausführungsform eignet sich insbesondere zur Stimulation einer Paddel- oder Schlagbewegung der Beine, im Englischen als *flutter kick* benannt. Von den Erfindern wurde erkannt, dass die Stimulation zum *flutter kick* sich ausgezeichnet für ein Schwimmtraining auf FES-Basis eignen. Im Gegensatz zu beispielsweise einer Froschbewegung der Beine kann deutlich zuverlässiger eine Fortbewegung und ein spürbarer Trainingserfolg erzielt werden.

Erfindungsgemäß ist die Vorrichtung dadurch gekennzeichnet, dass die Steuereinrichtung konfiguriert ist zur Stimulation einer Schwimmbewegung, bevorzugt einer zyklischen Paddelbewegung der Beine.

In einer bevorzugten Ausführungsform der Erfindung ist die Vorrichtung dadurch gekennzeichnet, dass die Steuereinrichtung konfiguriert ist durch mindestens zwei Elektroden eine zyklisch abwechselnde Stimulation beider Beine zu bewirken, wobei die zyklisch abwechselnde Stimulation bevorzugt mit einer Frequenz zwischen 0.2 Hz und 5 Hz erfolgt. Eine zyklische abwechselnde Stimulation meint, dass der Stimulator zunächst die elektrischen Pulse für die Muskelpartie eines Beines appliziert, um dessen Streckung oder Beugung zu bewirken. Im Anschluss erfolgt eine Applikation geeigneter elektrischen Pulse des anderen Beines, sodass durch die abwechselnde zyklische Stimulation eine zyklische Paddelbewegung (*flutter kick*) auslöst.

Überraschenderweise hat es sich erwiesen, dass auch eine zyklisch abwechselnde Stimulation des *Musculus quadriceps femoris* oder der *ischiocrurale* Muskulatur jeweils eines Beines ausreicht, um eine periodische Paddelbewegung zu initiieren. Teilweise kann dies auf die Rückführbewegung aufgrund der Translation des Körpers im Wasser zurückgeführt werden. Als besonders vorteilhaft hat sich hierfür der *Musculus quadriceps femoris* erwiesen. Eine zyklisch abwechselnde Stimulation dieser Muskelpartie auf jeweils einem Bein führt zu einer harmonischen Schwimmbewegung.

Die Frequenz der Stimulation bezeichnet bevorzugt jene Frequenz mit der die Beine abwechselnd zur Streckung oder Beugung gebracht werden. Bei einer Frequenz von 1 Hz, kann beispielsweise eine Stimulation der Muskelpartie eines Beines für 0,5 Sekunden, eine Pause von 0,5 Sekunden folgen, während derer die Muskelpartie des anderen Beins stimuliert wird. Beide Beine werden bei einem derartigen Stimulationsmuster zyklisch abwechselnd mit einer Frequenz von 1 Hz stimuliert.

Es kann jedoch auch besonders bevorzugt sein den *Musculus quadriceps femoris und* die *ischiocrurale* Muskulatur am selben Beines zyklisch abwechselnd zu stimulieren.

In einer bevorzugten Ausführungsform ist die Vorrichtung dadurch gekennzeichnet, dass die Vorrichtung mindestens vier Elektroden umfasst sowie Befestigungsmittel zur Positionierung jeweils mindestens zweier Elektroden auf der Haut jeweils eines Beines eines Menschen oberhalb des *Musculus quadriceps femoris* und der ischiokruralen Muskulatur.

Hierbei ist die Steuereinrichtung bevorzugt so konfiguriert, dass durch die jeweils mindestens zwei Elektroden pro Bein eines Menschen eine zyklisch abwechselnde Stimulation der beiden antagonistischen Muskelpartien und der beiden Beine erfolgt. Verschiedene Stimulationsmuster können hierzu angewandt werden. Durch die erhöhte Flexibilität lässt sich das Bewegungsmuster besonders gut an die individuellen Bedürfnisse der Nutzer anpassen, um angenehme Schwimmbewegungen zu realisieren.

In einer weiteren bevorzugten Ausführungsform kann die Vorrichtung so konfiguriert sein, dass die Stimulation des *Musculus quadriceps femoris* oder der ischiokrurale Muskulatur durch eine Stimulation *Musculus gluteus maximus* ergänzt wird. Der *Musculus gluteus maximus* (lat. für "größter Gesäßmuskel") bewirkt eine Streckung im Hüftgelenk und Stabilisierung des Oberschenkels, wodurch die Schwimmbewegung weiter unterstützt wird.

Offenbart wird auch eine Vorrichtung, die mindestens zwei Elektroden sowie Befestigungsmittel zur Positionierung der Elektroden auf der Haut eines Beines eines Menschen oberhalb des *Musculus quadriceps femoris,* der ischiokrurale Muskulatur, bevorzugt des *Musculus biceps femoris,* und/oder der Unterschenkelmuskulatur. Durch die Bereitstellung zweier Elektroden über den vorgenannten Muskelpartien kann vorteilhafterweise eine Untergruppe der Muskelpartien so stimuliert werden, dass eine Gangbewegung des Beines unterstützt wird.

Hierbei ist es daher bevorzugt, dass die Steuereinrichtung zur Stimulation einer Gangbewegung konfiguriert ist. Dem Fachmann sind eine Reihe von Stimulationsmustern zur Stimulation einer Gangbewegung außerhalb des Wassers bekannt. Überraschenderweise können diese Stimulationsmuster weitestgehend auch unter Wasser eingesetzt werden und zum gewünschten Erfolg führen.

Für die Erzeugung einer Gangbewegung im Wasser können Hüftstrecker (ischiokrurale Muskulatur), Kniestrecker (*Musculus quadriceps femoris*) oder Kniebeuger (*Musculus biceps femoris*) sowie Muskeln des Unterschenkels zyklisch stimuliert werden. Besonders bevorzugt ist es alle Muskelpartien umfassend Hüftstrecker, Kniestrecker oder -beuger sowie Muskeln des Unterschenkels zyklisch zu stimulieren. Je nach Bedarf kann aber auch nur eine Untermenge dieser Muskel durch Stimulation aktiviert werden. Bei Patienten mit einer Hemiparese reicht es i.d.R. aus nur das betroffene Bein zu unterstützen. Die Initiierung des Schrittes kann optional durch die schmerzhafte Stimulation der Fußsohle erfolgen. Zur Stabilisierung des Rumpfes kann eine zyklische oder dauerhafte Stimulation am Rumpf zum Einsatz kommen. Eine Stimulation der oberen Extremitäten kann den Gang und das Gleichgewicht im Wasser des Weiteren unterstützen. Alle Muskeln werden in bestimmten Gangphasen aktiviert. Geeignete Muster lassen sich z.B. aus EMG-Ableitungen Gesunder beim Gehen im Wasser bestimmen. Diese Muster sind bevorzugt durch die Steuereinrichtung fest vorgegeben, sodass der Patient seine Aktivität daran anpasst. Alternativ kann der Patient oder ein Therapeut die Stimulation manuell, z.B. mittels eines Handschalters bzw. Bediengerätes mit Gangphasen/-ereignissen des Patienten synchronisieren.

In einer bevorzugten Ausführungsform ist die Vorrichtung dadurch gekennzeichnet, dass diese eine Bekleidungsstück umfasst, in welchem die mindestens eine Elektrode derart positioniert vorliegt, sodass beim Tragen des Bekleidungsstückes durch einen Menschen die Elektroden auf der Haut eines Beines bevorzugt oberhalb des *Musculus quadriceps femoris* und/oder der ischiokrurale Muskulatur vorliegt, wobei es besonders bevorzugt ist, dass in dem Bekleidungsstück mindestens zwei Elektroden derart positioniert vorliegen, sodass beim Tragen des Bekleidungsstückes durch einen Menschen die Elektroden auf der Haut jeweils eines Beines bevorzugt oberhalb des *Musculus quadriceps femoris* und/oder der ischiokrurale Muskulatur vorliegen. Diese Ausführungsform ist besonders zur Stimulation einer Schwimmbewegung, bevorzugt einer zyklischen Paddelbewegung der Beine geeignet.

Offenbart wird auch eine Vorrichtung die dadurch gekennzeichnet, dass diese ein Bekleidungsstück umfasst, die Vorrichtung Befestigungsmittel zur Positionierung der mindestens einen Elektrode auf der Haut eines Beines eines Menschen oberhalb des *Musculus quadriceps femoris,* der ischiokruralen Muskulatur, des *Musculus biceps femoris* und/oder der Unterschenkelmuskulatur umfasst, wobei es besonders bevorzugt dass die Vorrichtung mindestens zwei Elektroden umfasst sowie Befestigungsmittel zur Positionierung der mindestens zwei Elektroden auf der Haut jeweils eines Beines eines Menschen oberhalb des *Musculus quadriceps femoris,* der ischiokrurale Muskulatur, des *Musculus biceps femoris* und/oder der Unterschenkelmuskulatur. Diese Vorrichtung ist insbesondere zur Stimulation einer Gangbewegung geeignet.

Die Ausführungsformen einer Elektrode auf der Haut eines Beines eines Menschen sind besonders geeignet für halbseitig gelähmte Probanden. Mittels des FES kann eine Paddelbewegung des eingeschränkten Beines wirkungsvoll stimuliert und mit der Bewegung des gesunden Beines synchronisiert werden.

Die Ausführungsformen zweier Elektroden auf der Haut jeweils eines Beines eines Menschen sind besonders geeignet für beidseitig gelähmte Probanden.

Bei der Verwendung der Vorrichtung zu Sport- oder Trainingszwecken im Freizeitbereich, also für Probanden ohne körperliche Einschränkungen, können je nach Trainingsbedarf beide Varianten vorteilhafterweise eingesetzt werden.

Das Bekleidungsstück kann eine Hose oder auch ein Schwimmanzug mit zusätzlichen Ärmeln für die Arme sein. Bevorzugt wird durch das Bekleidungsstück jedoch mindestens das Gesäß und die Oberschenkel abgedeckt, sodass eine sichere und angenehme Positionierung der Elektroden auf der *Musculus quadriceps femoris* oder der ischiokrurale Muskulatur vorgenommen werden kann. Im Falle einer gewünschten Stimulation der Unterschenkelmuskulatur wird das Bekleidungsstück bevorzugt auch diese Muskelpartien abdecken.

Die relative Positionierung der Elektroden über den gewünschten Muskelpartien in dem Bekleidungsstück ist durch die Anatomie des Menschen vorgegeben. Bei der Positionierung handelt es sich mithin um eine standardisierte Angabe, welche ein Fachmann ohne Weiteres auf Basis der Größe und Form eines Bekleidungsstückes festlegen kann. Die Positionierung der Elektroden kann durch Einnähen, Kleben oder anderen permanenten oder lösbaren Verbindungen zwischen Elektroden und dem Bekleidungsstück erfolgen. Die Ausführungsform der Erfindung erlaubt ein besonders anwenderfreundliches zügiges Anlegen, welches noch dazu besonders zuverlässig eine Positionierung der Elektroden auf den gewünschten Muskelpartien sicherstellt.

In einer weiteren bevorzugten Ausführungsform ist die Vorrichtung dadurch gekennzeichnet, dass diese neben den vorgenannten Elektroden mindestens zwei weitere Elektroden sowie Befestigungsmittel zur Positionierung einer Elektrode oberhalb des Rückenmarkes und einer Gegenelektrode seitlich oder am Bauch eines Menschen umfasst und die Steuereinrichtung konfiguriert ist, durch die beiden weiteren Elektrode eine Rückenmarksstimulation zu bewirken. Für eine transkutane Rückmarkstimulation liefern insbesondere Pulsenergien unter Wasser von 1.5 mJ bis 3 mJ ausgezeichnete Ergebnisse. Die transkutane Rückenmarkstimulation wirkt zum einen spastiklösend, schmerzunterdrückend und zum anderen durchblutungsfördernd, wodurch sich die Muskulatur der Probanden entspannt. Die transkutane Rückenmarkstimulation unterstützt daher eine Stimulation der Muskelbewegung, bspw. des *Musculus quadriceps femoris* oder der ischiokrurale Muskulatur, und führt zu flüssigen Bewegungsmustern und hohem Wohlbefinden.

In einer weiteren bevorzugten Ausführungsform ist die Vorrichtung dadurch gekennzeichnet, dass diese Auftriebskörper oder Schwimmgewichte umfasst. Auftriebskörper, auch als Schwimmkörper bekannt, sind aufgrund der Verdrängung von Wasser nach dem archimedischen Prinzip selbstständig schwimmfähig. D.h. ihr eigenes Gewicht ist geringer, als das Gewicht von Wasser desselben Volumens. Schwimmkörper hingegen zeichnen sich dadurch aus, dass ihr Gewicht höher ist als das Gewicht von Wasser desselben Volumens.

Durch Anbringung der Auftriebskörper am Körper oder Extremitäten des Probanden kann ein FES-Schwimmtraining vorteilhafterweise unterstützt werden. Die Dimensionierung kann zu diesem Zweck vom Fachmann gewählt werden. Zur Anbringung der Schwimmgewichte oder Auftriebskörper kann die Vorrichtung geeignete Befestigungsmittel wie beispielsweise Manschetten umfassen.

In einer weiteren bevorzugten Ausführungsform ist die Vorrichtung dadurch gekennzeichnet, dass diese mindestens einen Sensor sowie Befestigungsmittel zur Anbringung des Sensors an einer Extremität oder dem Rumpf umfasst, wobei der Sensor die Muskelaktivität und/oder die Bewegung der Extremität oder des Rumpfes misst und die Steuereinrichtung basierend auf den Messergebnissen die elektrischen Pulse generiert.

Die Bewegungsmessung der Extremität kann eine Bestimmung der Translation, Neigung oder Beschleunigung umfassen. Geeignete Sensoren sind beispielsweise Gyroskope, Beschleunigungssensoren u.a. piezoelektrisch oder kapazitiv, insbesondere auch als mikroelektro-mechanische Systeme (MEMS) oder Kombinationen dieser. Ganz besonders bevorzugt sind inertiale Messeinheiten (*inertial measurement unit IMU*), welche eine Kombination von Gyroskopen und Beschleunigungssensoren enthalten, um eine umfassende Abbildung der Bewegung des Rumpfes oder der Extremität zu erhalten.

Für die Messung der Muskelaktivität können Sensoren verwendet werden, wie sie im Fachbereich der Elektromyographie (EMG) bekannt sind. Die Elektromyographie ist eine Technik, die der Aufzeichnung von myoelektrischen Signalen dient. Myoelektrische Signale werden durch physiologische Zustandsvariationen der Muskelfasermembran generiert. Dabei geht es insbesondere um die willkürliche Muskelaktivierung von funktionellen Bewegungen. Im Stand der Technik sind zur Ableitung der Muskel-Aktionspotentiale Oberflächen-EMGs und intramuskuläres (Nadel- und Feindraht-) EMGs bekannt. Für die beschriebene Vorrichtung sind Oberflächen-EMGs besonders bevorzugt und führen zu keinerlei Beeinträchtigung der Bewegung. Die abgeleiteten Signale werden über Verstärker verstärkt und zur Auswertung an einen Analog-Digital-Umsetzer (ADU) übergegeben. Hierzu eignet sich beispielsweise der ADS1292 von Texas Instruments. Dieser besitzt zwei Eingangskanäle. Es ist somit möglich an zwei verschiedenen Muskeln gleichzeitig zu messen. Für Ausführungsformen in denen mehr als an zwei Muskeln eine Aktivität zu messen ist, wird bevorzugt ein ADU mit entsprechend Kanalbestückung gewählt. Für den ADU sind Spannungsauflösung von mehr als 8 bit, bevorzugt mehr als 16 bit, besonders bevorzugt 24 Bit oder mehr bevorzugt. Eine hohe Auflösung ermöglicht einen besseren Signal-Rauschabstand, wodurch die Muskelaktivität genauer bestimmt werden kann. Der bevorzugte ADU ADS1292 basiert auf einer Delta-Sigma-Modulation bei 125 kHz, womit es möglich ist, die Muskelaktivität mit einer maximalen Frequenz von 8 kHz abzutasten. Dies vereinfacht die Dimensionierung des dafür nötigen Anti-Aliasing-Filters. Das Bauteil besitzt eine variable Anzahl von Abtastwerten pro Sekunde und es ist möglich Daten zwischen 125 und 8000 Abtastwerten pro Sekunde auszugeben. Im Sensor ist ein Right-Leg-Drive (RLD) integriert. Dieser nutzt eine zusätzliche Elektrode, um das Netzbrummen zu unterdrücken.

Durch die Messung der Muskelaktivität oder Bewegung des Rumpfes oder einer Extremität ist es möglich eine besonders synchrone Schwimmbewegung zu erreichen. Durch Bewegungssensoren z.B. einer IMU erfolgt eine direkte Messung der Bewegung der Körperteile. Durch Messung der Muskelaktivität kann auch zusätzlich oder unabhängig von der tatsächlich ausgeführten Bewegung deren Intention bestimmt und für eine synchrone Stimulation genutzt werden.

Für die Paddelbewegung ist es vorteilhaft die FES der Beine mit Kraulbewegungen der Arme zu synchronisieren, um die Körperlage im Wasser zu stabilisieren, um Energieverluste zu vermeiden und ein effektives Schwimmen zu gewährleisten. Zu diesem Zweck können die Sensoren an den Armen angebracht werden und dort deren Bewegung, z.B. mittels eines IMU, aufzuzeichnen. Die Auswertung der Daten kann in der Steuereinrichtung selbst oder einer Datenverarbeitungseinrichtung erfolgen, wobei eine möglichst simultane Auswertung bevorzugt ist, um das Stimulationsmuster nahezu simultan an die Armbewegung anzupassen. Überraschenderweise liefert auch eine Messung des Rumpfes zu diesem Zweck ausgezeichnete Ergebnisse. Wird mit Hilfe einer IMU der Rollwinkel des Rumpfes bestimmt, kann eine FES des *Musculus quadriceps femoris* oder der ischiokruralen Muskulatur zur Erzeugung einer Paddelbewegung der Beine besonders zuverlässig mit der Kraulbewegung der Arme synchronisiert werden. Ausgezeichnete Ergebnisse werden erreicht, wenn die Stimulation der Muskelpartie eines Beines initiiert wird, sobald der Neigungswinkel des Rumpfes mehr als 10°, bevorzugt mehr als 15° beträgt und gleichzeitig für die Rumpfbewegung eine Winkelgeschwindigkeit von mehr als 30°/s, besonders bevorzugt mehr als 40°/s festgestellt wird.

In einer bevorzugten Ausführungsform der Erfindung ist die Vorrichtung dadurch gekennzeichnet, dass die Vorrichtung ein wasserdichtes Bedienelement aufweist, welches eine Steuerung der Stimulationsintensität und -muster der elektrischen Pulse erlaubt. Bevorzugt kann das Bedienelement am Körper mitgeführt werden, sodass der Proband selbst die Stimulationsintensität und -frequenz der elektrischen Pulse einstellen kann. Mit der Stimulationsintensität ist bevorzugt die Stromstärke, Pulsbreite und Frequenz der elektrischen Pulse während einer Stimulation gemeint. Je höher die Stromstärke, Pulsbreite und Frequenz der elektrischen Pulse desto größer ist die Stimulationsintensität, d.h. desto kraftvoller wird ein Muskel angeregt, desto schneller wird dieser jedoch auch ermüden. Mit dem Stimulationsmuster ist bevorzugt das Muster gemeint, welches dem gewünschten Bewegungsmuster zu Grunde liegt. Beispielsweise kann es sich hierbei um eine Frequenz für eine zyklische abwechselnde Stimulation der Beine während einer Paddelbewegung handeln. Es kann auch bevorzugt sein, dass durch das Bediengerät der Typ eines Bewegungsmusters, beispielsweise einer Schwimmbewegung ausgewählt werden kann und durch Spezifikation der Stimulationsfrequenz oder -intensität die konkrete Ausführung des jeweiligen Bewegungsmusters einstellbar ist.

Das Bediengerät erlaubt somit eine optimale Berücksichtigung individueller Bedürfnisse, welche auch vom Tagesempfinden oder der Tagesform abhängen können.

In einer bevorzugten Ausführungsform der Erfindung ist die Vorrichtung dadurch gekennzeichnet, dass der Stimulator eine Kommunikationsschnittstelle für ein Smart Device, bevorzugt ein Smartphone, eine Smartwatch und / oder ein Tablet aufweist.

Als Kommunikationsschnittstellen eignen sich sowohl Kabelschnittstellen (z.B. USB Schnittstelle) oder eine kabellose Kommunikationsschnittstelle, beispielsweise auf Basis von WLAN, Bluetooth oder andere Funkstandards. Die Kommunikationsschnittstelle ist bevorzugt in der Steuereinrichtung integriert und verfügt entsprechend bevorzugt über eine Empfänger- und Sendereinheit, welche den drahtlosen Austausch über Kommunikationsprotokolle ermöglichen.

Die Einbindbarkeit mobiler Endgeräte oder Smart Devices, wie eines Smartphone, einer Smartwatch und/oder ein Tablets über Kommunikationsschnittstellen erhöht die Bedienfreundlichkeit sowie die Möglichkeiten, komplexe Stimulationsmuster an jeweilige individuelle Bedürfnisse anzupassen. So können in dem Smart Device Daten über Trainingseinheiten aufgezeichnet werden. Auch ist das Aufstellen oder Abgleichen mit Trainingsplänen möglich, sodass automatisiert Vorschläge für Stimulationsmuster oder - intensitäten unterbreitet werden können. Hierfür können auch weitere Daten, wie durch das Smart Device aufgezeichnete biomedizinische Daten (Herzfrequenz, Fitness), einbezogen werden.

In einer bevorzugten Ausführungsform betrifft die Erfindung ein System umfassend eine erfindungsgemäße Vorrichtung oder eine bevorzugte Ausführungsform davon sowie ein Smart Device, sowie ein Datenverarbeitungsprogramm, welches auf dem Smart Device installiert vorliegt, dadurch gekennzeichnet, dass das Datenverarbeitungsprogramm eine Steuerung der Stimulationsfrequenz oder -intensität erlaubt. Das Datenverarbeitungsprogramm ist bevorzugt eine Anwendungssoftware, kurz App.

Neben der Auswahl und Steuerung der Stimulationsfrequenz oder -intensität kann die App bevorzugt auch Daten über vergangene Trainingseinheiten aufzeichnen und ggf. mit erstellten Trainingsplänen abgleichen.

Die Erfindung betrifft weiterhin die Verwendung einer erfindungsgemäßen Vorrichtung oder eines Systems oder bevorzugten Ausführungsformen davon zur Erzeugung einer sensorischen Stimulation oder einer Muskelbewegung im Wasser durch elektrische Stimulation von Nerven und/oder Muskeln.

In einer bevorzugten Ausführungsform betrifft die Erfindung ein Verfahren umfassend

- Bereitstellung einer erfindungsgemäßen Vorrichtung oder eines Systems oder bevorzugten Ausführungsformen, sowie
- Erzeugung einer sensorischen Stimulation oder einer Muskelbewegung im Wasser durch elektrische Stimulation von Nerven und/oder Muskeln mittels Anwendung der Vorrichtung oder des Systems

In einer bevorzugten Ausführungsform betrifft die Erfindung zudem ein Verfahren zur sensorischen Stimulation oder Erzeugung einer Muskelbewegung im Wasser durch elektrische Stimulation von Nerven und/oder Muskeln, umfassend die Schritte

- Bereitstellung einer Vorrichtung umfassend einen Stimulator mit einer Spannungsquelle und einer Steuereinrichtung, mindestens eine Elektrode zur Befestigung an einer Haut, und mindestens ein Kabel, welches den Stimulator mit der Elektrode verbindet, wobei der Stimulator, die mindestens eine Elektrode und das mindestens eine Kabel wasserdicht ausgebildet sind und die Steuereinrichtung konfiguriert ist, an der mindestens einen Elektrode elektrische Pulse zur Übertragung auf die Haut zu erzeugen

- Erzeugung von elektrischen Pulsen auf der Haut zur sensorischen Stimulation oder Erzeugung einer Muskelbewegung im Wasser

Der Fachmann erkennt, dass die offenbarten bevorzugten technischen Merkmale der Vorrichtung oder des Systems gleichermaßen für dessen Verwendung und daraus ableitbarer Verfahren gilt und dort dieselben vorteilhaften Wirkungen entfalten. Für die Vorrichtung wurde beispielsweise offenbart, dass diese bevorzugt mindestens zwei Elektroden zur Anbringung oberhalb des *Musculus quadriceps femoris* und/oder der ischiokrurale Muskulatur umfasst, wobei es besonders bevorzugt ist, dass die Steuereinrichtung zur zyklisch abwechselnden Stimulation von Muskelpartien beider Beine konfiguriert ist.

In einer weiteren bevorzugten Ausführungsform betrifft die Erfindung ein Verfahren zur sensorischen Stimulation oder Erzeugung einer Muskelbewegung im Wasser durch elektrische Stimulation von Nerven und/oder Muskeln, umfassend die Schritte

- Bereitstellung einer erfindungsgemäßen Vorrichtung, wobei die Vorrichtung mindestens zwei Elektroden umfasst sowie Befestigungsmittel zur Positionierung jeweils mindestens einer Elektrode auf der Haut jeweils eines Beines eines Menschen oberhalb des *Musculus quadriceps femoris* und/oder der ischiokrurale Muskulatur, die Steuereinrichtung konfiguriert ist durch die mindestens zwei Elektroden eine zyklisch abwechselnde Stimulation beider Beine zu bewirken, wobei die zyklisch abwechselnde Stimulation bevorzugt mit einer Frequenz zwischen 0.5 Hz und 5 Hz erfolgt

- Positionierung jeweils mindestens einer Elektrode auf der Haut jeweils eines Beines eines Menschen oberhalb des *Musculus quadriceps femoris* und/oder der ischiokruralen Muskulatur

- zyklisch abwechselnde Stimulation beider Beine, wobei die zyklisch abwechselnde Stimulation bevorzugt mit einer Frequenz zwischen 0.5 Hz und 5 Hz erfolgt.

Wie für die Ausführungsform der Vorrichtung offenbart, erlaubt auch das daraus ableitbare Verfahren eine besonders effektive Erzeugung einer Paddelbewegung (*flutter kick*) der Beine.

In einer bevorzugten Ausführungsform der Erfindung betreffen die Verwendungen und Verfahren ausschließlich den Freizeit- oder Sportbereich und bevorzugt ein Bewegungstraining für Probanden, welche nicht bewegungseingeschränkt sind. Hierbei entfalten die Verwendung und Verfahren besondere Trainingserfolge, nicht nur hinsichtlich der Steigerung von Muskelkraft und Ausdauer, sondern zudem in Bezug auf das Erlernen oder Optimieren von Bewegungsmuster, welche angenehm durch die FES-Anwendungen vermittelt werden können.

## DETAILLIERTE BESCHREIBUNG

Im Folgenden soll die Erfindung an Hand von Abbildungen und Beispielen näher erläutert werden, ohne auf diese beschränkt zu sein.

Kurzbeschreibung der Abbildungen

Fig. 1      Schematische Darstellung einer bevorzugten Ausführungsform der Vorrichtung

Fig. 2      Schematische Darstellung einer bevorzugten Ausführungsform des Stimulators

Fig. 3      Schematische Darstellung einer bevorzugten Ausführungsform einer wasserdichten Elektrode

Fig. 4      Schematische Darstellung einer bevorzugten Ausführungsform einer wasserdichten Steckverbindung zur Verkabelung der Elektrode mit dem Stimulator

Fig. 5      Illustration eines bevorzugten Stimulationsmusters zur Anregung des *Musculus quadriceps femoris*

Fig. 6      Experimentelle Daten einer Kniestreckung und -beugung durch Stimulation des *Musculus quadriceps femoris und* der ischiokruralen Muskulatur

Fig. 7      Unterwasseraufnahmen einer stimulierten Kraulbewegung eines querschnittsgelähmten Probanden

Fig. 8      Schematische Darstellung zur Illustration einer bevorzugten Messung der Bewegung des Rumpfes

Fig. 9      Schematische Darstellung einer Zustandsmaschine zur Regulation der Stimulation in Abhängigkeit einer Messung der Rumpfbewegung

Fig. 10      Experimentelle Daten einer Kraulbewegung unter Stimulation des *Musculus quadriceps femoris*

Detaillierte Beschreibung der Abbildungen

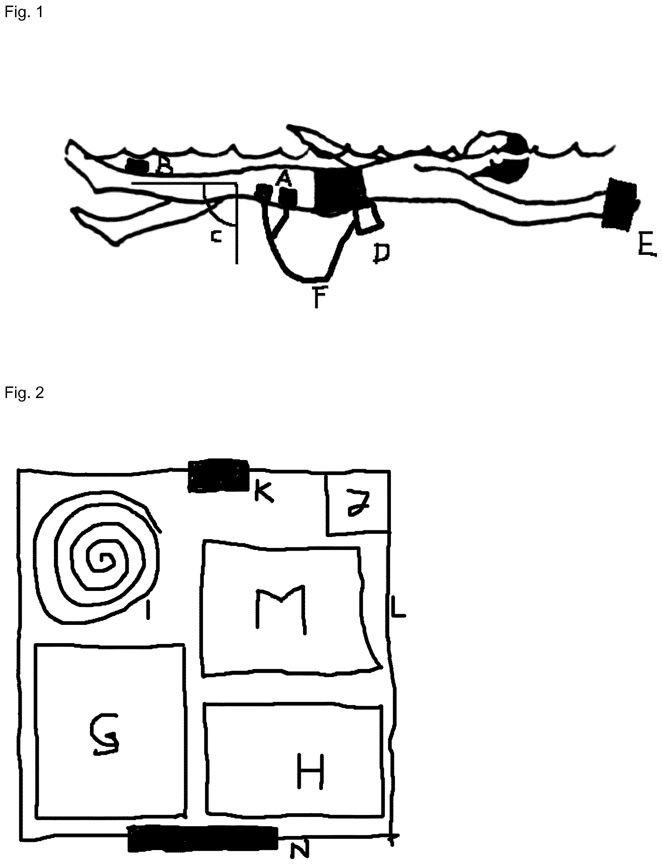

Figur 1 zeigt eine schematische Darstellung einer bevorzugten Ausführungsform der Vorrichtung. Wie in Figur 1 dargestellt, umfasst die Vorrichtung einen wasserdichten Stimulator mit integrierter Steuereinrichtung (D) der am Körper während des Schwimmens getragen wird und über ein oder mehrere wasserdichte Kabel (F) und eine oder mehrere wasserdichte Elektroden (A) mit dem Körper verbunden ist. Gleichzeitig sind am Unterschenkel und/oder Oberschenkel oder anderen Körperteilen Sensoren (B) zur Messung der Orientierung, Bewegung und/oder Muskelaktivität angebracht, die für die Steuerung der Stimulation verwendet werden können. Die Daten werden dabei über ein Kabel oder kabellos an den Stimulator (D) oder an ein Smart Device (E) übertragen. Durch Erzeugung von Stromimpulsen, die über das Kabel (F) und die Elektroden (A) an die Extremitäten weitergeleitet werden, kann eine funktionale Bewegung der Extremität erzeugt werden, die einen Vortrieb des Körpers im Wasser erzeugt. Zur Konfiguration der Stimulation und zur Variation der Stimulationsintensität kann ein Smart Device (E) (Smartphone / Smartwatch / Tablet) verwendet werden, welches über eine kabellose Kommunikation Daten mit dem Stimulator austauscht.

Figur 2 zeigt eine schematische Darstellung einer bevorzugten Ausführungsform des Stimulators. Wie in Figur 2 dargestellt, besteht der Stimulator dabei aus einer Hochspannungsquelle (H), einer Batterie (G), einer kabellosen Ladevorrichtung (I), einer kabellosen Kommunikationsvorrichtung (J), einem wasserdichten Konnektor für die Stimulationskabel (K), einem wasserdichten Gehäuse (L), einem Demultiplexer (M) sowie einem wasserdichten Bedienelement (N). Die im Stimulator vorhandene integrierte Steuereinrichtung wird in der Figur nicht gezeigt, kann aber beispielsweise ein Mikroprozessor sein.

Figur 3 zeigt eine schematische Darstellung einer bevorzugten Ausführungsform einer wasserdichten Elektrode. Die bevorzugten wasserdichten Elektroden umfassen ein leitfähiges flexibles Element mit einer Oberseite und einer Unterseite, einem Konnektor im Kontakt mit dem leitfähigen flexiblen Element zur Herstellung einer elektrischen Verbindung mit einem Stimulator, eine nicht-leitende flexiblen Folie, welche über der Oberseite des leitfähigen flexiblen Elements angeordnet ist, wobei die nicht-leitfähige Folie eine größere Abmessung als das leitfähigen flexible Element aufweist, sodass eine Überlappung entsteht, sowie bevorzugt einen mittigen Kleber zur Verbindung der nicht-leitenden flexiblen Folie mit dem leitfähiges flexiblen Element. Durch die Wasserdichtheit der Elektroden wird die weitere Funktion der Elektroden nach Baden, Duschen und Schwimmen gewährleistet. Die funktionale Verwendung der Elektroden im Wasser, d.h. die Applizierung elektrischer Impulse, würde aufgrund eines nicht wasserdichten Konnektors bzw. Steckverbindung für das Kabel beeinträchtigt. Für die Verwendung zur FES unter Wasser wurde daher die wasserdichte Klebefolie, Tegaderm von 3M, über der Verbindung von Elektrode und Kabel aufgebracht.

Figur 4 zeigt eine schematische Darstellung einer bevorzugten Ausführungsform einer wasserdichten Steckverbindung zur Verkabelung der Elektrode mit dem Stimulator. Dargestellt ist dies für einen 2 mm Stecker, wobei auch andere Stecker genutzt werden können. Der Stecker ist über eine Silikonleitung mit dem Stimulator verbunden. Um die Verbindung zwischen Elektrode und Kabel wasserdicht gestalten, wird einen Silikonschlauch verwandt. Weiterhin sind die Standardstecker der kommerzielle erhältlichen FES Stimulatoren in der Regel nicht wasserdicht. Somit kann Wasser auch von hinten in die Verbindung eindringen. Durch eine permanente Verklebung mittels Schrumpfschlauch oder Heißkleber kann auch diese Verbindungsstelle abgedichtet werden.

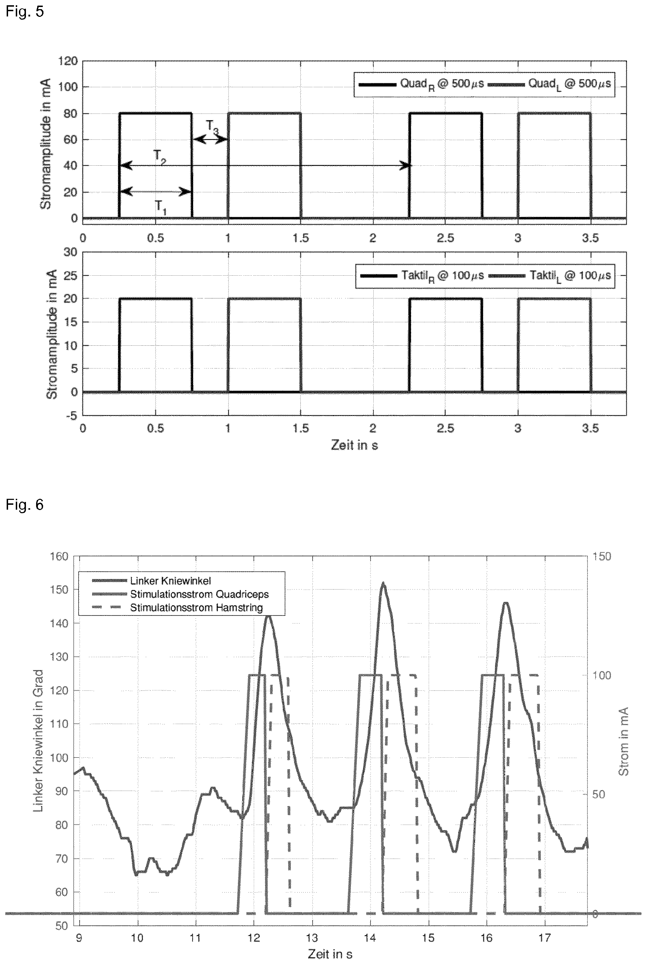

Figur 5 zeigt ein bevorzugtes Gate Signal für ein Stimulationsmuster zur Anregung des *Musculus quadriceps femoris.* T1 bezeichnet die Stimulationsdauer für einen Muskel, T2 die Periodendauer zwischen zwei Stimulationen des Quadriceps und T3 die Periodendauer zwischen einer zyklisch abwechselnden Stimulation des Quadrizeps des linken und rechten Beines. T1, T2 und T3 können an die Schwimmtechnik des Probanden angepasst werden Darüber hinaus können in gleicher Weise auch noch weitere Muskeln wie die ischiokrurale Muskulatur (Hamstring, Kniebeuger) oder des *Musculus gluteus maximus* (Hüftstrecker) stimuliert werden.

Figur 6 zeigt experimentelle Daten von Kniestreckung und -beugung durch Stimulation des *Musculus quadriceps femoris* und der ischiokruralen Muskulatur. Auf Basis des gezeigten Stimulationsmusters konnten drei aufeinanderfolgende Knieausschläge erzeugt werden.

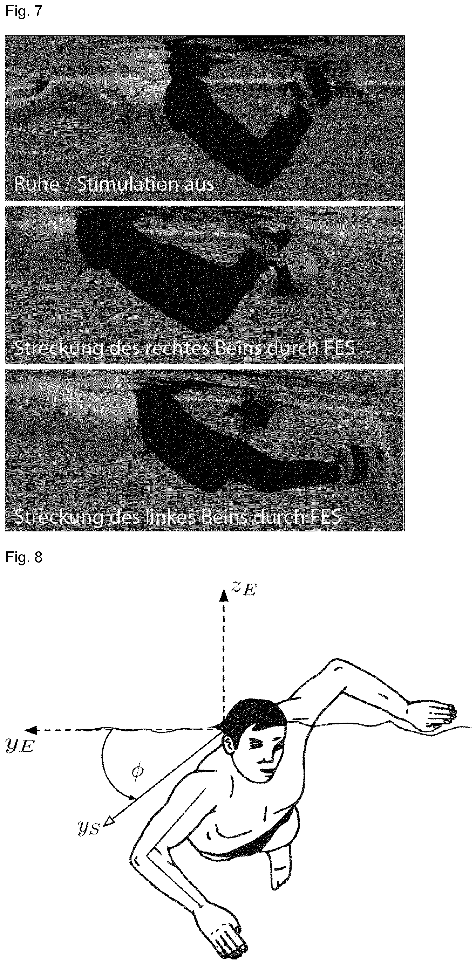

Figur 7 zeigt Unterwasseraufnahmen einer stimulierten Kraulbewegung eines querschnittsgelähmten Probanden (Th5, ASIA scale A). Zur Unterstützung der Kraulbewegung wurden Auftriebskörper an den Füßen angebracht und eine antisynchrone Stimulation des linken und rechten *Musculus quadriceps femoris* und der ischiokruralen Muskulatur durchgeführt. Durch eine Videoanalyse können experimentelle Daten über die Bewegung mit dem Stimulationsmuster korreliert werden (vgl. Figur 6).

Figur 8 zeigt eine schematische Darstellung einer bevorzugten Messung der Bewegung des Rumpfes. Hierzu wird ein IMU Sensor auf dem Rumpf des Probanden angebracht und der Rollwinkel $\varphi$ des Rumpfes bestimmt, wobei $z_E$ die z-Achse des Weltrahmens ist und $z_S$ und $y_S$ die z- und y-Achse des intrinsischen Sensorrahmens sind.

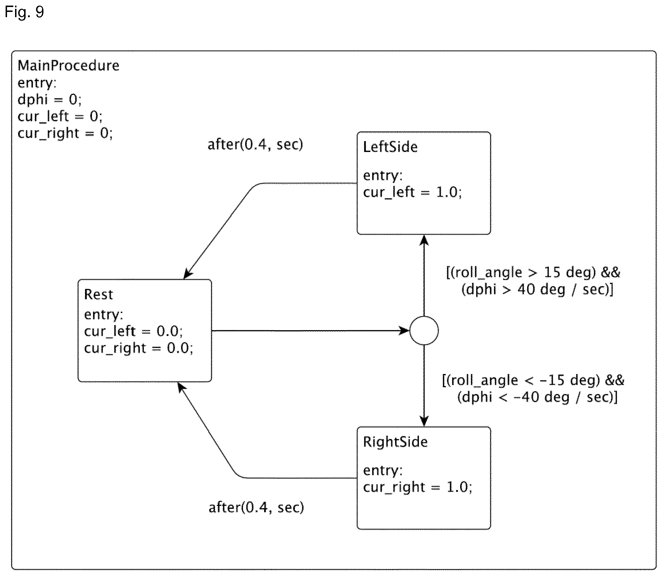

Figur 9 zeigt eine schematische Darstellung einer Zustandsmaschine zur Regulation der Stimulation in Abhängigkeit von der gemessenen Bewegung des Rumpfes. Die Zustandsmaschine ist in Stateflow implementiert, wobei jeder Eintrag eine singuläre Aktion definiert, die in Abhängigkeit vom Zustand für jeden Zeitindex k ausgeführt wird.

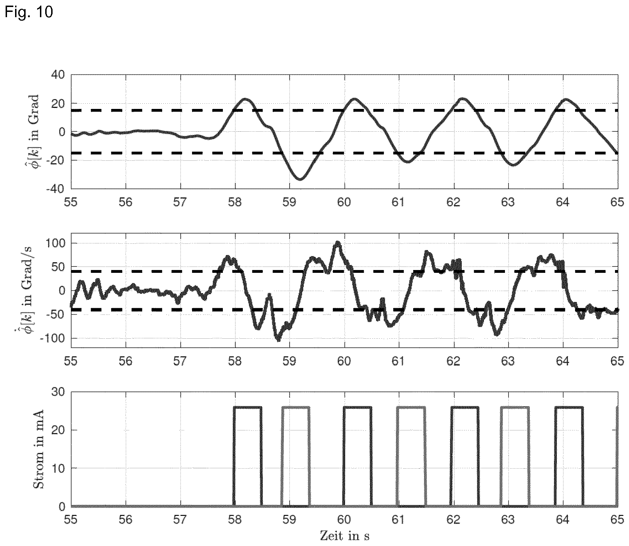

Figur 10 zeigt experimentelle Daten einer Kraulbewegung durch Stimulation des *Musculus quadriceps femoris.* Die experimentellen Daten wurden mit einer Abtastfrequenz T = 100 Hz für einen gesunden Probanden aufgenommen, der eine Kraulbewegung seiner Arme und seines Rumpfes ausführt, während die entsprechende Muskelpartie Musculus quadriceps femoris stimuliert wird (Pulsfrequenz f = 25 Hz, Pulsbreite von 300μs).

## Beispiele

*Beispiel 1 - Stimulationsmuster zur Erzeugung einer Paddelbewegung der Beine*

Um die beste Schwimmtechnik und Stimulationseinstellung zu finden wurden mehrere Vorversuche mit einem querschnittgelähmten Probanden unter ärztlicher Aufsicht durchgeführt. Die Stimulation des Gluteus Maximus wurde nicht vorgenommen, da es für den Probanden schwierig ist, die Elektroden ohne Hilfe zu platzieren. Als Ergebnis wurde entschieden, dass die Kraultechnik verwendet werden sollte in Kombination mit FES. Außerdem haben sich Schwimmkörper an den Beinen als nützlich erwiesen, da sie dafür sorgen, dass das Knie nach einer Streckung automatisch wieder in die Beugung geht.

In Fig. 7 sind drei Fotos in der Sagittalebene dargestellt, welche die verschiedenen Zustände der Stimulation zeigen. Während dieses Tests wurde der Proband gebeten, nicht mit seinen Armen zu schwimmen, um eine stabile Position für die Winkelaufzeichnung zu bekommen. Während des Versuchs wurden Unterwasser-Videodaten mit einer wasserdichten Kamera aufgenommen. Unter Verwendung der Videodaten wurde der linke Kniewinkel gemessen wie in Fig. 6 gezeigt. In Fig. 6 ist darüber hinaus die zyklische Stimulation des Quadriceps und Hamstrings zu sehen, was zu einer Paddelbewegung der Beine führt. In Kombination mit der Kraulbewegung der Arme kann so eine effiziente Schwimmbewegung erzeugt werden.

*Beispiel 2 - Synchronisation einer Stimulation der Beine mit den Rumpfbewegungen*

Um die Synchronisation der Beinbewegungen mit den Armbewegungen zu adressieren, wurde eine Vorrichtung und eine Methode entwickelt, die den Rollwinkel und/oder - winkelgeschwindigkeit des Rumpfes verwendet, um die Stimulation der Beine auszulösen. Der Rollwinkel des Rumpfes ist definiert als der Winkel zwischen der medio-lateralen Achse des Rumpfes und der horizontalen Ebene, wie in Fig. 5 dargestellt. Beim Schwimmen mit der Kraultechnik variiert dieser Winkel typischerweise periodisch im Bereich von ± 50 ° für professionelle Schwimmer und im Bereich von ± 30 ° für unerfahrene Schwimmer (cf. (Callaway et al., 2009; Bächlin et al., 2009)).

Ein Inertialsensor (IMU) ist an der Rückseite des Rumpfes derart angebracht, dass die intrinsische x-Achse des IMU mit der Längsachse des Rumpfes ausgerichtet ist und die intrinsische y-Achse des IMU mediolateral zur rechten Seite des IMU zeigt. Unter diesen Umständen kann der Rollwinkel über das folgende Verfahren berechnet werden.

In einem ersten Schritt wird die Orientierung des IMU aus der gemessenen Beschleunigung und der Winkelgeschwindigkeit ermittelt. Zu diesem Zweck wird der von Seel und Ruppin (2017) vorgeschlagene Algorithmus verwendet, der zu jedem Zeitindex *k* ein Quaternion $^S_{\mathcal{E}}q[k]$ liefert. Dieses Quaternion beschreibt die Orientierung des intrinsischen Sensorkoordinatensystems S des IMU in Bezug auf ein festes Referenzkoordinatensystem E mit vertikaler z-Achse.

Der angewandte Algorithmus ist modular und die von Seel und Ruppin (2017) beschriebenen Magnetometer-basierte Korrektur wird nicht verwendet, da die Azimuth-Information nicht benötigt wird. Die y-Achse des IMU in Sensorkoordinaten ist gegeben durch $^S_S\mathbf{y} = [0\ 1\ 0]^T$. Die entsprechenden Koordinaten im Referenzkoordinatensystem werden erhalten durch

$$\begin{bmatrix} 0 \\ ^S_{\mathcal{E}}\mathbf{y} \end{bmatrix} = {}^S_{\mathcal{E}}q[k] \otimes \begin{bmatrix} 0 \\ ^S_S\mathbf{y} \end{bmatrix} \otimes {}^S_{\mathcal{E}}q^{-1}[k], \quad (1)$$

wobei $\otimes$ die Quaternion-Multiplikation bezeichnet. Ebenso kann die gemessene Winkelgeschwindigkeit $^Sg$. in Bezugskoordinaten ausgedrückt werden durch

$$\begin{bmatrix} 0 \\ ^{\mathcal{E}}\mathbf{g} \end{bmatrix} = {}^S_{\mathcal{E}}q[k] \otimes \begin{bmatrix} 0 \\ ^S\mathbf{g} \end{bmatrix} \otimes {}^S_{\mathcal{E}}q^{-1}[k]. \quad (2)$$

Der Winkel zwischen der y-Achse des IMU und der vertikalen z-Achse des Referenzkoordinatensystems ist gleich dem Rumpfrollwinkel plus 90 °/s. Daher können wir nur mit der z-Komponente von $^S_{\mathcal{E}}\mathbf{y}$ den Rumpfrollwinkel bestimmen

$$\phi[k] = \arccos(^{\mathcal{E}}q[k]_z) - \frac{\pi}{2}. \quad (3)$$

In ähnlicher Weise wird die zeitliche Ableitung des Rumpfwinkels erhalten, indem die Winkelgeschwindigkeit $_{\mathcal{E}}g$. auf die Rollachse des Rumpfes projiziert wird, die senkrecht zu $^S_S\mathbf{y}$ und $^{\mathcal{E}}_{\mathcal{E}}\mathbf{z}$ ist:

$$\dot{\phi}[k] = {}_{\mathcal{E}}\mathbf{g} \cdot \left( \frac{^{\mathcal{E}}_{\mathcal{E}}\mathbf{z} \times {}^S_S\mathbf{y}}{\|^{\mathcal{E}}_{\mathcal{E}}\mathbf{z}\|_2 \|^S_S\mathbf{y}\|_2} \right). \quad (4)$$

Nach Sanders et al. (2017) und Deschodt et al. (1999) spielt der Beinschlag beim Kraulschwimmen eine Hauptrolle, um den Körper in einer stromlinienförmigen Position zu halten. Erfahrene Schwimmer realisieren mehrere Beinschläge während einer Armbewegung. Die Art der Synchronisation hängt vom Können des Schwimmers ab, da erfahrene Schwimmer während einer Armbewegung mehrere Beinschläge ausführen. Bei einer langsamen Schwimmbewegung ist es vorteilhaft die Kniestreckung jeder Seite mit der Streckung des kontralateralen Arms zu synchronisieren. Um die Rollwinkel-getriggerte Stimulation zu realisieren, wurde eine Zustandsmaschine (Fig. 5) konstruiert, welche basierend auf der Sensorinformation eine Stimulationsphase von 0.4 s startet, sobald der Rumpfwinkel 15 ° bei einer Winkelgeschwindigkeit von mehr als 40°/s übersteigt.

Mit dieser Zustandsmaschine wurde ein Experiment mit einem gesunden Probanden auf einer gepolsterten Liege durchgeführt, bei der der Rumpf über den Tisch hinausragt, um freie Schulterbewegungen zu ermöglichen. Der IMU-Sensor wurde mit einem Klettband zwischen den Schulterblättern befestigt. Zur Stimulation wurde der gleiche Stimulator wie für das Schwimmexperiment verwendet, aber nur der linke und der rechte Quadrizeps wurden stimuliert.

In Fig. 10 sind der Rollwinkel, die Geschwindigkeit und die resultierenden Stimulationsströme des Experiments dargestellt. Der Proband wurde gebeten, eine Kraulbewegung mit seinen Armen und seinem Rumpf durchzuführen. Wann immer die Stimulationskriterien erfüllt waren, wurde der entsprechende Quadrizepsmuskel stimuliert.

### Beispiel 3 - Traininaserfolae durch Schwimmtrainina

Zur Untersuchung, ob es technisch machbar ist, bei bis zu 10 Probanden mit Lähmung der unteren Extremitäten nach spinalem Trauma eine Schwimmbewegung durch Elektrostimulation im Wasser zu erzeugen, wird eine Studie durchgeführt.

Die Probanden sind über 18 Jahre alt und weisen eine Lähmung der unteren Extremitäten nach spinalem Trauma auf.

Die Studie umfasst eine Land- und eine Schwimmphase.

Während der Landphase führt jeder der 10 Probanden ein vierwöchiges FES Fahrradtraining durch. Dies umfasst mindestens drei Trainingseinheiten pro Woche von mindestens 30 Minuten auf einem RehaMove FES Fahrrad-Ergometer. Am Beginn und Ende dieser Trainingsphase wird der Oberschenkeldurchmesser und die maximale Fahrradleistung aufgezeichnet. Das FES-Radfahrtraining während der Landphase erfolgt, um eine definierte Muskelmasse der gelähmten Beine für den Schwimmversuch aufzubauen.

Während der Schwimmphase wird das FES-Fahrradfahren auf höchstens zwei Mal pro Woche reduziert.

Danach beginnt ein Schwimmtraining sowohl mit FES, als auch ohne FES. Die Schwimmbewertung bestehen aus einem strikten ABBA-Muster, um den Effekt der Ermüdung zu eliminieren.

Zudem werden die Schwimmversuche mit oder ohne FES durch jeweils eine Pause von 5 min getrennt. Während eines Schwimmversuchs (mit oder ohne FES) wird der Proband aufgefordert so schnell wie möglich eine Bahn zu schwimmen, wobei die Zeit dafür ermittelt wird. Am Anfang und Ende jedes Assessments wird die Spastizität über einen Modified Ashworth Scale-Test ermittelt.

Zudem füllt jeder Proband einen Fragebogen über sein Wohlbefinden und die Nutzbarkeit des Schwimmtrainings aus.

Die Studie zeigt:

a) Die Schwimmgeschwindigkeit mit FES Unterstützung erhöht sich gegenüber nicht unterstütztem Schwimmen

b) Das allgemeine Wohlbefinden der Probanden verbessert sich signifikant durch das Schwimmtraining mittels FES Unterstützung

c) Die Nutzer zeigen eine hohe Akzeptanz ggü. der Technologie, sodass eine gute Compliance zu erwarten ist.

### Beispiel 4 - Kombination einer zyklischen Stimulation mit einer transkutanen Rückenmarksstimulation

Bei zwei querschnittgelähmten Proband (Asia A) einer Pilotstudie wurde zusätzlich zur zyklischen Stimulation einer Paddelbewegung eine transkutanen Rückenmarksstimulation durchgeführt.

Für die Rückenmarkstimulation wurde eine 5x5 cm Elektrode auf dem Rücken oberhalb der Wirbelableitungen T11 und T12 platziert und eine elektrisch verbundene Gegenelektrode (5 x 9cm oder größer) auf dem Bauch. Die Stimulationsfrequenz sollte im Bereich 30 bis 50 Hz liegen. Bei den Versuchen wurde 40 Hz als Frequenz gewählt. Es wurden insbesondere biphasische Impulse mit 1 ms Zeitdauer pro Phase ohne eine Pause zwischen den Phasen appliziert. Die Stimulationsamplitude wurde für die Rückenmarkstimulations in den ersten Tests der Pilotstudie so gewählt, dass eine sichtbare Tonuserhöhung im Rumpf und in den Beinen auftritt, welche zu einer Streckung des Körpers führt.

Folgende Effekte einer zyklischen Stimulation der Beine zugleich mit einer Rückenmarksstimulation wurden beobachtet:

1) Die Körperlage beim Schwimmen verbessert sich wesentlich, da der Tonus der Rumpf-, Hüft- und Beinmuskulatur erhöht ist und der Körper somit besser gesteckt ist. Dies führte zu einer Steigerung der Schwimmgeschwindigkeit gegenüber einer reinen Stimulation der Beine um 30 Prozent. Die Schwimmgeschwindigkeit war um 30 Prozent größer bei der Kombination von Rückenmark- und Beinstimulation gegenüber dem Schwimmen ohne Stimulation.

2) Weiterhin wurde eine nahezu vollständige Reduktion einer zuvor vorhandenen Spastik für bis zu 4 Stunden nach einem 30-minutigen Schwimmtraining mit aktiver Rückenmarkstimulation festgestellt. Bei alleiniger Stimulation der Beine war diese in dem Maße nicht feststellbar.

Es wird darauf hingewiesen, dass verschiedene Alternativen zu den beschriebenen Ausführungsformen der Erfindung verwendet werden können, um die Erfindung auszuführen und zu der erfindungsgemäßen Lösung zu gelangen. Die erfindungsgemäße Vorrichtung, das System sowie deren Verwendung in den beschrieben Verfahren beschränken sich in ihren Ausführungen somit nicht auf die vorstehenden bevorzugten Ausführungsformen. Vielmehr ist eine Vielzahl von Ausgestaltungsvarianten denkbar, welche von der dargestellten Lösung abweichen können. Ziel der Ansprüche ist es, den Schutzumfang der Erfindung zu definieren. Der Schutzumfang der Ansprüche ist darauf gerichtet, die erfindungsgemäße Vorrichtung, das System deren bevorzugte Verwendungen bzw. Verfahren sowie äquivalente Ausführungsformen von diesen abzudecken.

## LITERATUR

Axelgaard, J. (2004). Current-Controlling Electrode with Adjustable Contact Area. US 6,745,082.

Axelgaard, J. (2010). Moisture Resistant Electrode with Edge Protection. US 7,697,999.

Bächlin, M., Förster, K., and Tröster, G. (2009). SwimMaster: A Wearable Assistant for Swimmer. In Proceedings of the 11th international conference on Ubiquitous computing - Ubicomp '09, 215. ACM Press, New York, New York, USA.

Bromley, I. (2006). Tetraplegia and paraplegia : a guide for physiotherapists. Churchill Livingstone.

Brunelli, G. (2014). Topics in Paraplegia. fig 1. InTech, 1 edition.

Callaway, A.J., Cobb, J.E., and Jones, I. (2009). A Comparison of Video and Accelerometer Based Approaches Applied to Performance Monitoring in Swimming. International Journal of Sports Science & Coaching, 4(1), 139-153.

Deschodt, V.J., Arsac, L.M., and Rouard, A.H. (1999). Relative contribution of arms and legs in humans to propulsion in 25-m sprint front-crawl swimming. European Journal of Applied Physiology and Occupational Physiology, 80(3), 192-199.

Gibbons, R.S., Stock, C.G., Andrews, B.J., Gall, A., and Shave, R.E. (2016). The effect of FES-rowing training on cardiac structure and function: Pilot studies in people with spinal cord injury. Spinal Cord, 54(10), 822-829.

J. Jung, E. Chung, K. Kim, B.-H. Lee, and J. Lee (2014). The effects of aquatic exercise on pulmonary function in patients with spinal cord injury. J. Phys. Ther. Sci., vol. 26, no. 5, pp. 707-9.

Nash, M.S. (2005). Exercise as a health-promoting activity following spinal cord injury. Journal of Neurologie Physical Therapy, 29(2), 87-106.

Newham, D.J. and Donaldson, N.D.N. (2007). FES cycling. Acta Neurochirurgica, Supplementum, 97(97 PART 1), 395-402.

Sanders, R.H., Andersen, J.T., and Takagi, H. (2017). The Segmental Movements in Front Crawl Swimming. In Handbook of Human Motion, 1-15. Springer International Publishing, Cham.

Seel, T. and Ruppin, S. (2017). Eliminating the Effect of Magnetic Disturbances on the Inclination Estimates of Inertial Sensors. IFAC-PapersOnLine, 50(1), 8798- 8803.

Seifert, L., L'Hermette, M., Wattebled, L., Komar, J., Mell, F., Gomez, D., and Caritu, Y. (2011). Use of Inertial Central to Analyse Skill of Inter-Limb Coordination in Sport Activities. BIO Web of Conferences, 1, 1-4.

Seifert, L., Leblanc, H., Chollet, D., and Deligni'eres, D. (2010). Inter-limb coordination in swimming: Effect of speed and skill level. Human Movement Science, 29(1), 103-113.

Stevens, S.L., Caputo, J.L., Fuller, D.K., and Morgan, D.W. (2015). Effects of underwater treadmill training on leg strength, balance, and walking performance in adults with incomplete spinal cord injury. The Journal of Spinal Cord Medicine, 38(1), 91-101.

Tamburella, F., Scivoletto, G., Cosentino, E., and Molinari, M. (2013). Walking in Water and on Land After an Incomplete Spinal Cord Injury. American Journal of Physical Medicine & Rehabilitation, 92(10 Suppl 2), e4-e15.

Tawashy, A.E., Eng, J.J., Lin, K.H., Tang, P.F., and Hung, C. (2009). Physical activity is related to lower levels of pain, fatigue and depression in individuals with spinalcord injury: A correlational study. Spinal Cord, 47(4), 301-306.

Wiesener, C. and Schauer, T. (2017). The Cybathlon RehaBike: Inertial-Sensor-Driven Functional Electrical Stimulation Cycling by Team Hasomed. IEEE Robotics & Automation Magazine, 24(4), 49-57.

**Patentansprüche**

1. Vorrichtung zur Erzeugung einer Muskelbewegung im Wasser durch elektrische Stimulation von Nerven und/oder Muskeln, umfassend einen Stimulator (D) mit einer Spannungsquelle (H) und einer Steuereinrichtung, mindestens eine Elektrode (A) zur Befestigung an einer Haut, und mindestens ein Kabel (F), welches den Stimulator (D) mit der Elektrode (A) verbindet, wobei der Stimulator (D), die mindestens eine Elektrode (A) und das mindestens eine Kabel (F) wasserdicht ausgebildet sind und die Steuereinrichtung konfiguriert ist an der mindestens einen Elektrode (A) elektrische Pulse zur Übertragung auf die Haut zu erzeugen, wobei die Steuereinrichtung konfiguriert ist zur Stimulation einer Schwimmbewegung.

2. Vorrichtung gemäß dem vorherigen Anspruch **dadurch gekennzeichnet, dass** die mindestens eine Elektrode (A) mittels eines Sprühpflasters und/oder Sprühverbandes wasserdicht versiegelt wurde und/oder die Verbindung der mindestens eine Elektrode (A) mit dem mindestens einen Kabel (F) durch ein von einem Silikonschlauch umfassten Stecker erfolgt.

3. Vorrichtung nach einem der vorherigen Ansprüche, **dadurch gekennzeichnet, dass** die elektrischen Pulse mono- oder biphasisch sind und eine Energie von 10 mJ nicht überschreiten, wobei die Pulse bevorzugt eine Stromstärke zwischen 0-120 mA und eine Pulsbreite zwischen 0 und 1000 μs aufweisen.

4. Vorrichtung nach einem der vorherigen Ansprüche, **dadurch gekennzeichnet, dass** die Vorrichtung Befestigungsmittel zur Positionierung der mindestens einen Elektrode (A) auf der Haut eines Beines eines Menschen oberhalb des *Musculus quadriceps femoris* und/oder der ischiokrurale Muskulatur umfasst, wobei es besonders bevorzugt dass die Vorrichtung mindestens zwei Elektroden (A) umfasst sowie Befestigungsmittel zur Positionierung der mindestens zwei Elektroden (A) auf der Haut jeweils eines Beines eines Menschen oberhalb des *Musculus quadriceps femoris* und/oder der ischiokrurale Muskulatur.

5. Vorrichtung nach dem vorherigen Anspruch, **dadurch gekennzeichnet, dass** die Steuereinrichtung konfiguriert ist zur Stimulation einer zyklischen Paddelbewegung der Beine.

6. Vorrichtung nach dem vorherigen Anspruch, **dadurch gekennzeichnet, dass** die Steuereinrichtung konfiguriert ist durch mindestens zwei Elektroden (A), eine zyklisch abwechselnde Stimulation beider Beine zu bewirken, wobei die zyklisch abwechselnde Stimulation bevorzugt mit einer Frequenz zwischen 0.5 Hz und 5 Hz erfolgt.

7. Vorrichtung nach einem der vorherigen Ansprüche, **dadurch gekennzeichnet, dass** die Vorrichtung eine Bekleidungsstück umfasst, in welchem die mindestens eine Elektrode derart positioniert vorliegt, sodass beim Tragen des Bekleidungsstückes durch einen Menschen die Elektroden auf der Haut eines Beines bevorzugt oberhalb des *Musculus quadriceps femoris* und/oder der ischiokrurale Muskulatur vorliegt, wobei es besonders bevorzugt ist, dass in dem Bekleidungsstück mindestens zwei Elektroden (A) derart positioniert vorliegen, sodass beim Tragen des Bekleidungsstückes durch einen Menschen die Elektroden (A) auf der Haut jeweils eines Beines bevorzugt oberhalb des *Musculus quadriceps femoris* und/oder der ischiokrurale Muskulatur vorliegen.

8. Vorrichtung nach einem der vorherigen Ansprüche, **dadurch gekennzeichnet, dass** die Vorrichtung Auftriebskörper oder Gewichte zur Befestigung an den Extremitäten eines Menschen umfasst.

9. Vorrichtung nach einem der vorherigen Ansprüche 4-10, **dadurch gekennzeichnet, dass** die Vorrichtung mindestens zwei weitere Elektroden (A) sowie Befestigungsmittel zur Positionierung einer Elektrode (A) oberhalb des Rückenmarkes und einer Gegenelektrode (A) seitlich oder am Bauch eines Menschen umfasst und die Steuereinrichtung konfiguriert ist, durch die beiden weiteren Elektrode (A) eine Rückenmarksstimulation zu bewirken.

10. Vorrichtung nach einem der vorherigen Ansprüche, **dadurch gekennzeichnet, dass** die Vorrichtung mindestens einen Sensor (B) sowie Befestigungsmittel zur Anbringung des Sensors an einer Extremität oder dem Rumpf umfasst, wobei der Sensor (B) die Muskelaktivität und/oder die Bewegung der Extremität oder Rumpfes misst und die Steuereinrichtung basierend auf den Messergebnissen die elektrischen Pulse generiert.

11. Vorrichtung nach einem der vorherigen Ansprüche, **dadurch gekennzeichnet, dass** ein wasserdichtes Bedienelement aufweist, welches eine Steuerung der Stimulationsintensität und -frequenz der elektrischen Pulse erlaubt.

12. System umfassend eine Vorrichtung nach einem der vorherigen Ansprüche, ein smart device,

sowie ein Datenverarbeitungsprogramm, welches auf dem smart device installiert vorliegt,
**dadurch gekennzeichnet, dass**
das Datenverarbeitungsprogramm eine Stimulationsintensität und -frequenz der elektrischen Pulse erlaubt.

**Claims**

1. A device for generating a muscle movement in water through electrical stimulation of nerves and/or muscles, comprising a stimulator (D) with a voltage source (H) and a control device,
at least one electrode (A) for attachment on the skin, and at least one cable (F) connecting the stimulator (D) to the electrode (A), wherein the stimulator (D), the at least one electrode (A), and the at least one cable (F) are designed to be watertight, and the control device is configured to generate electrical pulses for transfer to the skin on the at least one electrode (A),
wherein the control device is configured to stimulate a swimming motion.

2. The device according to the previous claim
**characterized in that**
the at least one electrode (A) is sealed in a watertight fashion by means of a spray bottle, and/or
a spray adhesive, and/or
the at least one electrode (A) is connected to the at least one cable (F) by means of a plug surrounded by a silicone tube.

3. The device according to any one of the previous claims,
**characterized in that** the electrical pulses are mono- or biphasic, and do not exceed an energy of 10 mJ, wherein the pulses preferably have an amperage between 0-120 mA and a pulse width between 0 and 1,000 $\mu$s.

4. The device according to any one of the previous claims,
**characterized in that** the device comprises fastening means for positioning the at least one electrode (A) on the skin of a human leg above the *quadriceps femoris muscle* and/or the ischiocrural musculature, wherein it is particularly preferred that the device comprises at least two electrodes (A) as well as fastening means for positioning the at least two electrodes (A) on the skin of a respective human leg above the *quadriceps femoris muscle* and/or the ischiocrural musculature.

5. The device according to the previous claim,
**characterized in that** the control device is configured to stimulate a cyclic paddling movement of the legs.

6. The device according to the previous claim,
**characterized in that** the control device is configured by at least two electrodes (A) to effect cyclically alternating stimulation of either leg, the cyclically alternating stimulation preferably taking place at a frequency between 0.5 Hz and 5 Hz.

7. The device according to any one of the previous claims,
**characterized in that**
the device comprises a garment, wherein the at least one electrode is positioned such that when the garment is worn by a human, the electrodes are present on the skin of a leg, preferably above the *quadriceps femoris muscle,* and/or the ischiocrural musculature, wherein it is particularly preferred that at least two electrodes (A) are positioned in the garment, such that when the garment is worn by a human, the electrodes (A) are present on the skin of either leg, preferably above the *quadriceps femoris muscle* and/or the ischiocrural muscles.

8. The device according to any one of the previous claims,
**characterized in that**
the device comprises buoyancy elements or weights for attachment to the extremities of a human.

9. The device according to any one of the previous claims 4-8,
**characterized in that**
the device comprises at least two further electrodes (A), as well as fastening means for positioning an electrode (A) above the spinal cord and a counter electrode (A) laterally or on the human abdomen, and the control device is designed to effect spinal cord stimulation by means of the two further electrodes (A) .

10. The device according to any one of the preceding claims,
**characterized in that** the device comprises at least one sensor (B), as well as attachment means for attaching the sensor on an extremity or the torso, wherein the sensor (B) measures the muscle activity and/or movement of the extremity or torso, and the control device generates electrical pulses based on the results of the measurement.

11. The device according to any one of the preceding claims,
**characterized in that** it comprises a watertight control element allowing the stimulation intensity and frequency of the electrical pulses to be controlled.

12. A system comprising

a device according to any one of previous claims,
a smart device,
as well as a data processing program installed on
the smart device,
**characterized in that**
the data processing program allows a certain stimulation intensity and frequency of the electrical pulses.

**Revendications**

1. Dispositif de production d'un mouvement musculaire dans l'eau par stimulation électrique de nerfs et/ou de muscles, comprenant un stimulateur (D) avec une source de tension (H) et un dispositif de commande, au moins une électrode (A) pour la fixation sur une peau, et au moins un câble (F), lequel relie le stimulateur (D) à l'électrode (A), dans lequel le stimulateur (D), l'au moins une électrode (A) et l'au moins un câble (F) sont réalisés de manière étanche à l'eau et le dispositif de commande est configuré pour produire au niveau de l'au moins une électrode (A) des impulsions électriques pour la transmission sur la peau, dans lequel le dispositif de commande est configuré pour la stimulation d'un mouvement de natation.

2. Dispositif selon la revendication précédente **caractérisé en ce que** l'au moins une électrode (A) a été scellée étanche à l'eau au moyen d'un pansement à vaporiser et/ou d'un bandage à vaporiser et/ou la liaison de l'au moins une électrode (A) à l'au moins un câble (F) se fait par un connecteur entouré par un tuyau en silicone.

3. Dispositif selon l'une quelconque des revendications précédentes, **caractérisé en ce que** les impulsions électriques sont monophasées ou biphasées et ne dépassent pas une énergie de 10 mJ, dans lequel les impulsions présentent de préférence une intensité entre 0-120 mA et une largeur d'impulsion entre 0 et 1000 µs.

4. Dispositif selon l'une quelconque des revendications précédentes, **caractérisé en ce que** le dispositif comprend des moyens de fixation pour le positionnement de l'au moins une électrode (A) sur la peau d'une jambe d'une personne au-dessus du *musculus quadriceps femoris* et/ou de la musculature ischiocrurale, dans lequel il est particulièrement préféré que le dispositif comprend au moins deux électrodes (A) ainsi que des moyens de fixation pour le positionnement des au moins deux électrodes (A) sur la peau respectivement d'une jambe d'une personne au-dessus du *musculus quadriceps femoris* et/ou de la musculature ischiocrurale.

5. Dispositif selon la revendication précédente, **Caractérisé en ce que** le dispositif de commande est configuré pour la stimulation d'un mouvement de pale cyclique des jambes.

6. Dispositif selon la revendication précédente, **caractérisé en ce que** le dispositif de commande est configuré pour provoquer par au moins deux électrodes (A), une stimulation cycliquement alternée des deux jambes, dans lequel la stimulation cycliquement alternée se fait de préférence avec une fréquence entre 0,5 Hz et 5 Hz.

7. Dispositif selon l'une quelconque des revendications précédentes, **caractérisé en ce que** le dispositif comprend un vêtement, dans lequel l'au moins une électrode est positionnée de sorte que lors du port du vêtement par une personne, les électrodes sont sur la peau d'une jambe de préférence au-dessus du *musculus quadriceps femoris* et/ou de la musculature ischiocrurale, dans lequel il est particulièrement préféré qu'au moins deux électrodes (A) sont positionnées dans le vêtement de sorte que lors du port du vêtement par une personne les électrodes (A) sont sur la peau respectivement d'une jambe de préférence au-dessus du *musculus quadriceps femoris* et/ou de la musculature ischiocrurale.

8. Dispositif selon l'une quelconque des revendications précédentes, **caractérisé en ce que** le dispositif comprend des flotteurs ou des poids à fixer au niveau des extrémités d'une personne.

9. Dispositif selon l'une quelconque des revendications précédentes 4-8, **caractérisé en ce que** le dispositif comprend au moins deux autres électrodes (A) ainsi que des moyens de fixation pour le positionnement d'une électrode (A) au-dessus de la moelle épinière et d'une contre-électrode (A) latéralement ou sur le ventre d'une personne et le dispositif de commande est configuré pour provoquer une stimulation de la moelle épinière par les deux autres électrodes (A).

10. Dispositif selon l'une quelconque des revendications précédentes, **caractérisé en ce que** le dispositif comprend au moins un capteur (B) ainsi que des moyens de fixation pour le montage du capteur au niveau d'une extrémité ou du torse, dans lequel le capteur (B) mesure l'activité musculaire et/ou le mouvement de l'extrémité ou du torse et le dispositif de commande génère les impulsions électriques sur la base des résultats de mesure.

**11.** Dispositif selon l'une quelconque des revendications précédentes,
**caractérisé en ce que** présente un élément de service étanche à l'eau, lequel permet une commande de l'intensité de stimulation et de la fréquence de stimulation des impulsions électriques.

**12.** Système comprenant
un dispositif selon l'une quelconque des revendications précédente,
un dispositif intelligent,
ainsi qu'un programme de traitement de données, lequel est installé sur le dispositif intelligent,
**caractérisé en ce que**
le programme de traitement de données permet une intensité de stimulation et fréquence de stimulation des impulsions électriques.

Fig. 1

Fig. 2

Fig. 3

Fig. 4

Permanente (nicht-
lösbare) Anbringung

Nicht permanente
(lösbare) Anbringung

Fig. 5

Fig. 6

Fig. 7

Ruhe / Stimulation aus

Streckung des rechtes Beins durch FES

Streckung des linkes Beins durch FES

Fig. 8

Fig. 9

MainProcedure
entry:
dphi = 0;
cur_left = 0;
cur_right = 0;

after(0.4, sec)

LeftSide

entry:
cur_left = 1.0;

Rest
entry:
cur_left = 0.0;
cur_right = 0.0;

[(roll_angle > 15 deg) &&
(dphi > 40 deg / sec)]

[(roll_angle < -15 deg) &&
(dphi < -40 deg / sec)]

RightSide

entry:
cur_right = 1.0;

after(0.4, sec)

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9004955 A
- US 7697999 B2
- US 6745082 A
- US 7697999 B


**In der Beschreibung aufgeführte Nicht-Patentliteratur**

- **AXELGAARD, J.** *Current-Controlling Electrode with Adjustable Contact Area,* 2004
- **AXELGAARD, J.** *Moisture Resistant Electrode with Edge Protection,* 2010
- SwimMaster: A Wearable Assistant for Swimmer. **BÄCHLIN, M. ; FÖRSTER, K. ; TRÖSTER, G.** Proceedings of the 11th international conference on Ubiquitous computing - Ubicomp '09, 215. ACM Press, 2009
- **BROMLEY, I.** Tetraplegia and paraplegia : a guide for physiotherapists. Churchill Livingstone, 2006
- **BRUNELLI, G.** Topics in Paraplegia. 2014
- **CALLAWAY, A.J. ; COBB, J.E. ; JONES, I.** A Comparison of Video and Accelerometer Based Approaches Applied to Performance Monitoring in Swimming. *International Journal of Sports Science & Coaching,* 2009, vol. 4 (1), 139-153
- **DESCHODT, V.J. ; ARSAC, L.M. ; ROUARD, A.H.** Relative contribution of arms and legs in humans to propulsion in 25-m sprint front-crawl swimming. *European Journal of Applied Physiology and Occupational Physiology,* 1999, vol. 80 (3), 192-199
- **GIBBONS, R.S. ; STOCK, C.G. ; ANDREWS, B.J. ; GALL, A. ; SHAVE, R.E.** The effect of FES-rowing training on cardiac structure and function: Pilot studies in people with spinal cord injury. *Spinal Cord,* 2016, vol. 54 (10), 822-829
- **J. JUNG ; E. CHUNG ; K. KIM ; B.-H. LEE ; J. LEE.** The effects of aquatic exercise on pulmonary function in patients with spinal cord injury. *J. Phys. Ther. Sci.,* 2014, vol. 26 (5), 707-9
- **NASH, M.S.** Exercise as a health-promoting activity following spinal cord injury. *Journal of Neurologie Physical Therapy,* 2005, vol. 29 (2), 87-106
- **NEWHAM, D.J. ; DONALDSON, N.D.N.** FES cycling. *Acta Neurochirurgica, Supplementum,* 2007, vol. 97, 395-402
- The Segmental Movements in Front Crawl Swimming. **SANDERS, R.H. ; ANDERSEN, J.T. ; TAKAGI, H.** Handbook of Human Motion. Springer International Publishing, 2017, 1-15
- **SEEL, T. ; RUPPIN, S.** Eliminating the Effect of Magnetic Disturbances on the Inclination Estimates of Inertial Sensors. *IFAC-PapersOnLine,* 2017, vol. 50 (1), 8798-8803
- **SEIFERT, L. ; L'HERMETTE, M. ; WATTEBLED, L. ; KOMAR, J. ; MELL, F. ; GOMEZ, D. ; CARITU, Y.** Use of Inertial Central to Analyse Skill of Inter-Limb Coordination in Sport Activities. *BIO Web of Conferences,* 2011, vol. 1, 1-4
- **SEIFERT, L. ; LEBLANC, H. ; CHOLLET, D. ; DELIGNI'ERES, D.** Inter-limb coordination in swimming: Effect of speed and skill level. *Human Movement Science,* 2010, vol. 29 (1), 103-113
- **STEVENS, S.L. ; CAPUTO, J.L. ; FULLER, D.K. ; MORGAN, D.W.** Effects of underwater treadmill training on leg strength, balance, and walking performance in adults with incomplete spinal cord injury. *The Journal of Spinal Cord Medicine,* 2015, vol. 38 (1), 91-101
- **TAMBURELLA, F. ; SCIVOLETTO, G. ; COSENTINO, E. ; MOLINARI, M.** Walking in Water and on Land After an Incomplete Spinal Cord Injury. *American Journal of Physical Medicine & Rehabilitation,* 2013, vol. 92 (2), e4-e15
- **TAWASHY, A.E. ; ENG, J.J. ; LIN, K.H. ; TANG, P.F. ; HUNG, C.** Physical activity is related to lower levels of pain, fatigue and depression in individuals with spinalcord injury: A correlational study. *Spinal Cord,* 2009, vol. 47 (4), 301-306
- **WIESENER, C. ; SCHAUER, T.** The Cybathlon RehaBike: Inertial-Sensor-Driven Functional Electrical Stimulation Cycling by Team Hasomed. *IEEE Robotics & Automation Magazine,* 2017, vol. 24 (4), 49-57